(19) Europäisches Patentamt
European Patent Office
Office européen des brevets

(11) EP 4 253 384 A1

(12) **EUROPEAN PATENT APPLICATION**
published in accordance with Art. 153(4) EPC

(43) Date of publication:
**04.10.2023 Bulletin 2023/40**

(21) Application number: **21896948.3**

(22) Date of filing: **23.11.2021**

(51) International Patent Classification (IPC):
***C07D 487/04*** *(2006.01)* ***C07D 403/00*** *(2006.01)*

(52) Cooperative Patent Classification (CPC):
**C07D 403/00; C07D 487/04**

(86) International application number:
**PCT/CN2021/132330**

(87) International publication number:
**WO 2022/111447 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.11.2020 CN 202011326024**

(71) Applicant: **Haisco Pharmaceuticals Pte. Ltd.**
**Singapore 079903 (SG)**

(72) Inventors:
- **YI, Shixu**
  **Chengdu, Sichuan 611130 (CN)**
- **CHEN, Zengfei**
  **Chengdu, Sichuan 611130 (CN)**
- **PAN, Xusong**
  **Chengdu, Sichuan 611130 (CN)**
- **WANG, Wei**
  **Chengdu, Sichuan 611130 (CN)**
- **LI, Yanlin**
  **Chengdu, Sichuan 611130 (CN)**
- **HE, Yongyao**
  **Chengdu, Sichuan 611130 (CN)**

(74) Representative: **Hoffmann Eitle**
**Patent- und Rechtsanwälte PartmbB**
**Arabellastraße 30**
**81925 München (DE)**

(54) **METHOD FOR PREPARING BTK DEGRADING AGENT**

(57) A method for preparing a compound as represented by formula (I) and an intermediate thereof. The method has mild reaction conditions, does not involve a high-temperature and high-pressure reaction, has low-toxicity or non-toxicity raw materials, simple operation, a high reaction yield, a high product purity, convenient work-up, and good reproducibility, and is suitable for industrial production.

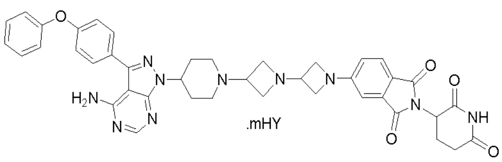

(I)

EP 4 253 384 A1

## Description

### Technical Field

The present invention relates to a method for preparing a compound as represented by formula (I) and an intermediate thereof.

### Background Art

Bruton's tyrosine kinase (BTK), a member of the Tec family of non-receptor protein tyrosine kinases, is a key regulator in the B cell antigen receptor (BCR) signalling pathway, and is distributed in the lymphatic system, hematopoietic system and blood system. BTK mutations may activate downstream signalling pathways in tumor cell proliferation, differentiation, angiogenesis, etc., which may lead to X-linked agammaglobulinemia, non-Hodgkin's lymphoma (NHL) and many B-cell malignancies, including chronic lymphocytic leukaemia (CLL), mantle cell lymphoma, and diffuse large B-cell lymphoma. As mainly expressed in B cells and myeloid cells, BTK is a target with relatively high targeting ability and safety.

PROTAC (proteolysis targeting chimera) molecules are a class of dual function compounds which are capable of binding to both targeted proteins and E3 ubiquitin ligases. This class of compounds can induce recognition of targeted proteins by proteasomes in a cell to cause the degradation of the targeted protein, which can effectively reduce the contents of the targeted proteins in the cell. By introducing a ligand capable of binding to various targeted proteins into the PROTAC molecules, it is possible to apply the PROTAC technology to the treatment of various diseases, and this technology has attracted extensive attention in recent years.

PCT/CN 2020/093455 discloses a BTK-Protac small molecule anti-tumour drug (as represented by the structure of compound 1 below), which is a triplet composed of a small molecule inhibitor targeting a BTK protein, a ligand for recruiting an E3 ubiquitin ligase, and a linker linking them. In one aspect, the anti-tumour drug can directly inhibit the activity of BTK by specifically binding BTK. In another aspect, the anti-tumour drug can induce the ubiquitination of BTK and degrade the BTK protein via the proteasome pathway, thereby blocking the transduction of the BCR signalling pathway and inhibiting the cell growth and proliferation of B-cell lymphoma. Therefore, the anti-tumour drug has dual effects on tumours.

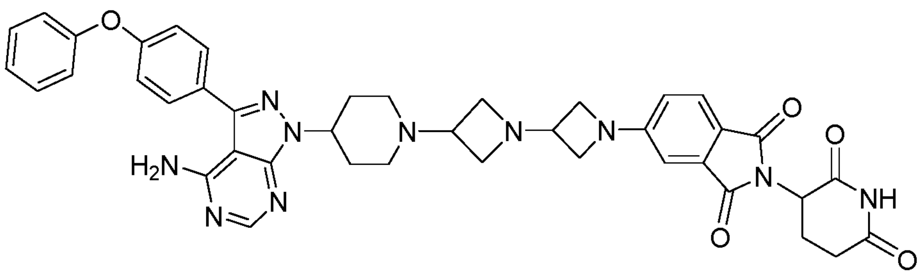

Compound 1

### Summary of the Invention

An objective of the present invention is to provide a method for preparing a compound as represented by formula (I) and an intermediate thereof. The method has mild reaction conditions, does not involve a high-temperature and high-pressure reaction, has low-toxicity or non-toxicity raw materials, simple operation, less by-products, a high intermediate product purity and convenient work-up. The whole process route has a good reproducibility and is suitable for industrial production.

The present invention relates to a method for preparing compound (II) by the following reaction formula:

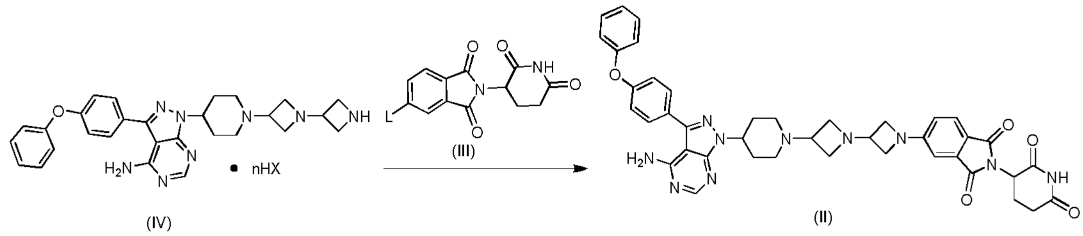


wherein L is selected from a trifluoromethanesulphonate group, F, Cl, Br, I,

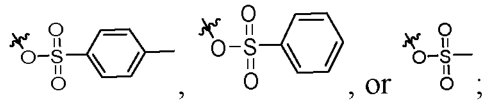
, , or ;

HX is selected from acetic acid, hydrochloric acid, sulphsulphuric acid, hydrobromic acid, hydroiodic acid or trifluoroacetic acid;

n is selected from 0, 1, 1.5, 2, 3 or 4;

and compound (IV) is reacted with compound (III) in the presence of an alkaline reagent and a solvent to obtain the compound (II).

In some embodiments of the method for preparing compound (II) in the present invention, when n = 0, i.e., the compound (IV) is in the form of a free base, the molar ratio of the alkaline reagent to the compound (IV) is ≤ 4.90 : 1, ≤ 4.85 : 1, or ≤ 4.80 : 1.

In some embodiments of the method for preparing compound (II) in the present invention, the alkaline reagent is selected from an organic amine reagent, preferably one or more of triethylamine, diethylamine or N,N-diisopropylethyl amine.

In some embodiments of the method for preparing compound (II) in the present invention, the alkaline reagent comprises an organic amine reagent, preferably one or more of triethylamine, diethylamine or N,N-diisopropylethylamine.

In some embodiments of the method for preparing compound (II) in the present invention, the solvent is selected from a polar aprotic solvent, preferably one or more of acetonitrile, N,N-dimethylformamide, N,N-diethylformamide, N,N-dimethylacetamide, dimethylsulphoxide or N-methyl-2-pyrrolidone.

In some embodiments of the method for preparing compound (II) in the present invention, the solvent comprises a polar aprotic solvent, preferably one or more of acetonitrile, N,N-dimethylformamide, N,N-diethylformamide, N,N-dimethylacetamide, dimethylsulphoxide or N-methyl-2-pyrrolidone.

In some embodiments of the method for preparing compound (II) in the present invention, the solvent is selected from dimethylsulphoxide.

In some embodiments of the method for preparing compound (II) in the present invention, the reaction is optionally performed at a temperature of 30°C-120°C, 60°C-110°C, or 80°C-100°C.

In some embodiments of the method for preparing compound (II) in the present invention, the molar ratio of the compound (III) to the compound (IV) is ≤ 10 : 1, preferably 1 : 1-3 : 1.

In some embodiments of the method for preparing compound (II) in the present invention, when n = 0, i.e., the compound (IV) is in the form of a free base, the molar ratio of the alkaline reagent to the compound (IV) is ≤ 5 : 1, and the reaction is optionally performed at a temperature of 60°C-110°C, 80°C-100°C, 85°C-95°C, or 85°C-90°C.

In some embodiments of the method for preparing compound (II) in the present invention, when n = 0, i.e., the compound (IV) is in the form of a free base, the alkaline reagent comprises N,N-diisopropylethylamine, the molar ratio of the alkaline reagent to the compound (IV) is ≤ 5 : 1, and the reaction is optionally performed at a temperature of 60°C-110°C, 80°C-100°C, 85°C-95°C, or 85°C-90°C.

In some embodiments of the method for preparing compound (II) in the present invention, when n = 1, 1.5, 2, 3 or 4, i.e., the compound (IV) is in salt form, the molar ratio of the alkaline reagent to the compound (IV) is optionally ≤ 10 : 1 or ≤ 6 : 1.

In some embodiments of the method for preparing compound (II) in the present invention, when n = 1, 1.5, 2, 3 or 4, i.e., the compound (IV) is in salt form, the molar ratio of the alkaline reagent to the compound (IV) is optionally 0.8 : 1-10 : 1, 1 : 1-10 : 1, 0.8 : 1-6 : 1, or 1 : 1-6 : 1.

In some embodiments of the method for preparing compound (II) in the present invention, after the reaction is completed, the reaction solution is subjected to a work-up to obtain the compound (II), wherein the work-up comprises layering the reaction solution, adding the lower layer to water for crystallization, filtering the resulting mixture, and slurrying

and filtering the filtrate.

In some embodiments of the method for preparing compound (II) in the present invention, after the reaction is completed, the reaction solution is subjected to a work-up to obtain the compound (II), wherein the work-up comprises adding the reaction solution to water for crystallization, filtering the resulting mixture, and slurrying and filtering the filtrate.

In some embodiments of the method for preparing compound (II) in the present invention, after the reaction is completed, the work-up comprises crystallizing or/and slurrying the compound (II).

In some embodiments of the method for preparing compound (II) in the present invention, after the reaction is completed, the work-up comprises crystallizing or/and slurrying the compound (II), wherein the solvent used comprises methanol or ethanol.

The method for preparing compound (II) described in the present invention has the following advantages: compound (IV) is in salt form, which provides a better stability; the method has a high reaction yield and produces less by-products and impurities; and the purification process is simple.

In some embodiments of the method for preparing compound (II) in the present invention, the compound (II) is reacted with HY to prepare compound (I),

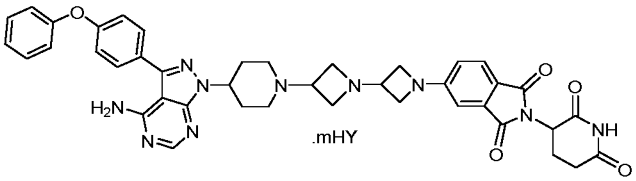

(I)

wherein HY is selected from a pharmaceutically acceptable salt, preferably fumaric acid, formic acid, acetic acid, butanedioic acid, hydrochloric acid, sulphuric acid, tartaric acid, p-methylbenzoic acid, methanesulphonic acid, malic acid, maleic acid and succinic acid; and
m is selected from 0.5, 1, 1.5, 2 or 3.

In some embodiments of the preparation of compound (I) in the reaction of compound (II) with HY in the present invention, the solvent in the reaction of the compound (II) with HY is selected from one of or a mixed solvent of two or more of an alkane solvent, a halogenated alkane solvent, an alcohol solvent, a ketone solvent, an ester solvent, an ether solvent, a nitrile solvent and water.

In some embodiments of the preparation of compound (I) in the reaction of compound (II) with HY in the present invention, the solvent in the reaction of the compound (II) with HY comprises one of or a mixed solvent of two or more of an alkane solvent, a halogenated alkane solvent, an alcohol solvent, a ketone solvent, an ester solvent, an ether solvent, a nitrile solvent and water.

In some embodiments of the preparation of compound (I) in the reaction of compound (II) with HY in the present invention, the solvent in the reaction of the compound (II) with HY is selected from one or more of dichloromethane, 1,2-dichloroethane, ethyl acetate, acetone, methanol, ethanol, ethylene glycol, polyethylene glycol, isopropanol, diethyl ether, tetrahydrofuran and water, preferably one or more of dichloromethane, methanol and water.

In some embodiments of the preparation of compound (I) in the reaction of compound (II) with HY in the present invention, the solvent in the reaction of the compound (II) with HY comprises one or more of dichloromethane, 1,2-dichloroethane, ethyl acetate, acetone, methanol, ethanol, ethylene glycol, polyethylene glycol, isopropanol, diethyl ether, tetrahydrofuran and water, preferably one or more of dichloromethane, methanol and water.

The present invention also relates to a method for preparing compound (IV) or compound (VI-1) by reaction formula (1) or (2) as follows:

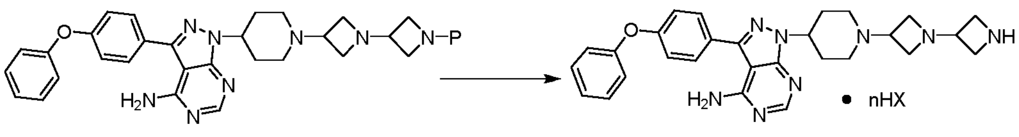

(V) (IV) (1)

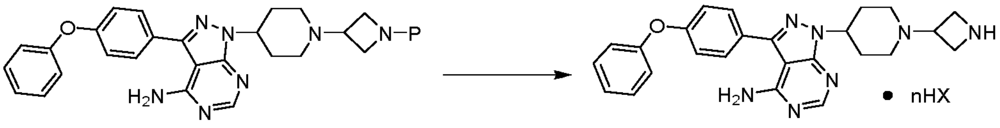

(VII) (VI-1) (2)

wherein P is selected from an amino protecting group, preferably tertbutoxycarbonyl, benzyloxycarbonyl, methoxycarbonyl, ethoxycarbonyl, p-toluenesulphonyl, trifluoroacetyl, triphenylmethyl and p-methoxybenzyl;
HX is selected from acetic acid, sulphuric acid, hydrobromic acid, hydroiodic acid or trifluoroacetic acid;
n is selected from 0, 1, 1.5, 2, 3 or 4;
compound (V) is reacted in the presence of an acidic reagent HX to obtain the compound (IV);
and compound (VII) is reacted in the presence of an acidic reagent HX to obtain the compound (VI-1).

In some embodiments of the preparation of compound (IV) by reaction formula (1) in the present invention, the reaction involves a solvent selected from a polar protic solvent, a polar aprotic solvent or a mixture thereof, preferably one or more of methanol, ethanol, water, dichloromethane, 1,2-dichloroethane, chloroform, carbon tetrachloride or tetrahydrofuran.

In some embodiments of the preparation of compound (IV) by reaction formula (1) in the present invention, the reaction involves a solvent, which comprises a polar protic solvent, a polar aprotic solvent or a mixture thereof, preferably one or more of methanol, ethanol, water, dichloromethane, 1,2-dichloroethane, chloroform, carbon tetrachloride or tetrahydrofuran.

In some embodiments of the preparation of compound (IV) by reaction formula (1) in the present invention, the reaction is optionally performed at a temperature of 0°C-60°C, 10°C-40°C, or 20°C-30°C.

In some embodiments of the preparation of compound (IV) by reaction formula (1) in the present invention, the work-up of the reaction comprises concentrating the reaction solution (such as concentration under reduced pressure or under normal pressure), then crystallizing or/and slurrying the resulting concentrate with an organic solvent, filtering the resulting mixture, and drying the filter cake.

In some embodiments of the preparation of compound (VI-1) by reaction formula (2) in the present invention, the solvent is selected from a polar protic solvent, a polar aprotic solvent or a mixture thereof, preferably one or more of methanol, ethanol, water, dichloromethane, 1,2-dichloroethane, chloroform, carbon tetrachloride or tetrahydrofuran.

In some embodiments of the preparation of compound (VI-1) by reaction formula (2) in the present invention, the solvent comprises a polar protic solvent, a polar aprotic solvent or a mixture thereof, preferably one or more of methanol, ethanol, water, dichloromethane, 1,2-dichloroethane, chloroform, carbon tetrachloride or tetrahydrofuran.

In some embodiments of the preparation of compound (VI-1) by reaction formula (2) in the present invention, the reaction is optionally performed at a temperature of 0°C-60°C, 10°C-40°C, or 20°C-30°C.

In some embodiments of the preparation of compound (VI-1) by reaction formula (2) in the present invention, the work-up of the reaction comprises concentrating the reaction solution under reduced pressure, then crystallizing or/and slurrying the resulting concentrate with an organic solvent, filtering the resulting mixture, and drying the filter cake.

The present invention also relates to a method for preparing compound (IV) or compound (VI-1) by reaction formula (3) or (4) as follows:

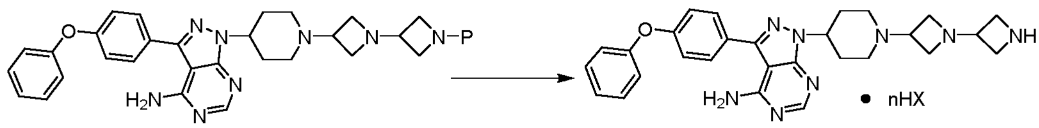

(V) (IV)

(3)

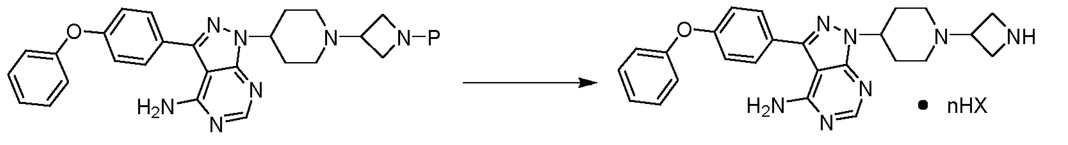

(VII) (VI-1)

(4)

wherein P is selected from an amino protecting group, preferably tertbutoxycarbonyl, benzyloxycarbonyl, methoxycarbonyl, ethoxycarbonyl, p-toluenesulphonyl, trifluoroacetyl, triphenylmethyl and p-methoxybenzyl;

HX is selected from hydrochloric acid;

n is selected from 0, 1, 1.5, 2, 3 or 4;

compound (V) is reacted in the presence of hydrochloric acid and a polar protic solvent to obtain the compound (IV); and

compound (VII) is reacted in the presence of hydrochloric acid and a polar protic solvent to obtain the compound (VI-1).

In some embodiments of the preparation of compound (IV) by reaction formula (3) in the present invention, the solvent is selected from a polar protic solvent, preferably one or more of methanol, ethanol and water, a polar aprotic solvent is optionally further added to the reaction, and the polar aprotic solvent is preferably one or more of dichloromethane, 1,2-dichloroethane, chloroform, carbon tetrachloride or tetrahydrofuran.

In some embodiments of the preparation of compound (IV) by reaction formula (3) in the present invention, the solvent comprises a polar protic solvent, preferably one or more of methanol, ethanol and water, a polar aprotic solvent is optionally further added to the reaction, and the polar aprotic solvent preferably comprises one or more of dichloromethane, 1,2-dichloroethane, chloroform, carbon tetrachloride or tetrahydrofuran.

In some embodiments of the preparation of compound (IV) by reaction formula (3) in the present invention, the reaction is optionally performed at a temperature of 0°C-60°C, 10°C-40°C, or 20°C-30°C.

In some embodiments of the preparation of compound (VI-1) by reaction formula (4) in the present invention, the solvent is selected from a polar protic solvent, preferably one or more of methanol, ethanol and water, a polar aprotic solvent is optionally further added to the reaction, and the polar aprotic solvent is preferably one or more of dichloromethane, 1,2-dichloroethane, chloroform, carbon tetrachloride or tetrahydrofuran.

In some embodiments of the preparation of compound (VI-1) by reaction formula (4) in the present invention, the solvent comprises a polar protic solvent, preferably one or more of methanol, ethanol and water, a polar aprotic solvent is optionally further added to the reaction, and the polar aprotic solvent preferably comprises one or more of dichloromethane, 1,2-dichloroethane, chloroform, carbon tetrachloride or tetrahydrofuran.

In some embodiments of the preparation of compound (VI-1) by reaction formula (4) in the present invention, the reaction is optionally performed at a temperature of 0°C-60°C, 10°C-40°C, or 20°C-30°C.

The present invention also relates to a method for preparing compound (V) or (VII) by the following reaction formulas:

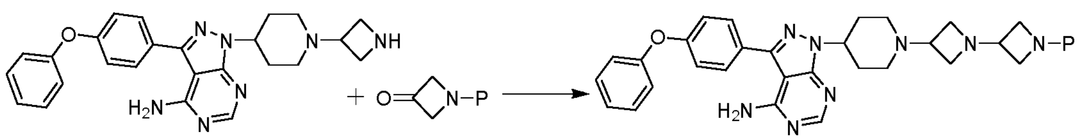

1a

(VI) (V)

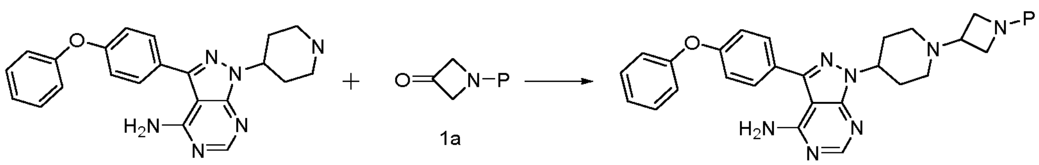

1a

(VIII) (VII)

wherein P is selected from an amino protecting group, preferably tertbutoxycarbonyl, benzyloxycarbonyl, methoxycarbonyl, ethoxycarbonyl, p-toluenesulphonyl, trifluoroacetyl, triphenylmethyl and p-methoxybenzyl;

compound (VI) is reacted with 1a in the presence of an acidic reagent and a reducing agent to obtain the compound (V); and

compound (VIII) is reacted with 1a in the presence of an acidic reagent and a reducing agent to obtain the compound (VII).

In some embodiments of the preparation of compound (V) or (VII) in the present invention, the desiccant is selected from one or more of anhydrous sodium sulphate, anhydrous magnesium sulphate, anhydrous calcium sulphate or a molecular sieve.

In some embodiments of the preparation of compound (V) or (VII) in the present invention, the desiccant comprises one or more of anhydrous sodium sulphate, anhydrous magnesium sulphate, anhydrous calcium sulphate or a molecular sieve.

In some embodiments of the preparation of compound (V) or (VII) in the present invention, compound (VI) is reacted with 1a in the presence of an acidic reagent, a desiccant and a reducing agent to obtain the compound (V); and compound (VIII) is reacted with 1a in the presence of an acidic reagent, a desiccant and a reducing agent to obtain the compound (VII).

In some embodiments of the preparation of compound (V) or (VII) in the present invention, the reaction is performed at a temperature of 0°C-40°C, preferably 20°C-40°C.

In some embodiments of the preparation of compound (V) or (VII) in the present invention, compound (VI) is reacted with 1a in the presence of an acidic reagent and a reducing agent, optionally with the addition of a desiccant, and then subjected to a work-up to obtain the compound (V), and the reaction is performed at a temperature of preferably 0°C-40°C, more preferably 20°C-40°C;

and compound (VIII) is reacted with 1a in the presence of an acidic reagent and a reducing agent, optionally with the addition of a desiccant, and then subjected to a work-up to obtain the compound (VII), and the reaction is performed at a temperature of preferably 0°C-40°C, more preferably 20°C-40°C.

In some embodiments of the preparation of compound (V) or (VII) in the present invention, the reaction involves a solvent selected from a polar aprotic solvent, preferably one or more of 1,2-dichloroethane, chloroform or dichloromethane.

In some embodiments of the preparation of compound (V) or (VII) in the present invention, the reaction involves a solvent, which comprises a polar aprotic solvent, preferably one or more of 1,2-dichloroethane, chloroform or dichloromethane.

In some embodiments of the preparation of compound (V) or (VII) in the present invention, the acidic reagent is optionally one or more of hydrochloric acid, acetic acid, formic acid, propionic acid, butyric acid, sulphuric acid, hydrobromic acid, hydroiodic acid or trifluoroacetic acid.

In some embodiments of the preparation of compound (V) or (VII) in the present invention, the acidic reagent comprises one or more of hydrochloric acid, acetic acid, formic acid, propionic acid, butyric acid, sulphuric acid, hydrobromic acid, hydroiodic acid or trifluoroacetic acid.

In some embodiments of the preparation of compound (V) or (VII) in the present invention, the reducing agent is selected from a boron reducing agent, preferably one or more of sodium borohydride, sodium triacetoxyborohydride, sodium triethylborohydride, sodium cyanoborohydride, potassium borohydride or lithium borohydride.

In some embodiments of the preparation of compound (V) or (VII) in the present invention, the reducing agent comprises a boron reducing agent, preferably one or more of sodium borohydride, sodium triacetoxyborohydride, sodium triethylborohydride, sodium cyanoborohydride, potassium borohydride or lithium borohydride.

In some embodiments of the preparation of compound (V) or (VII) in the present invention, the preparation method also comprises a work-up, wherein the work-up comprises: adjusting a reaction system to a neutral to weakly basic pH, extracting, and concentrating an organic phase to obtain the compound (V) or (VII).

In some embodiments of the preparation of compound (V) or (VII) in the present invention, the work-up further comprises crystallizing or/and slurrying the compound (V) or (VII) with a solvent, filtering, and drying a filter cake, and optionally, the solvent for the slurrying is preferably methyl tert-butyl ether or diethyl ether.

In some embodiments of the preparation of compound (V) or (VII) in the present invention, the work-up further comprises crystallizing or/and slurrying the compound (V) or (VII) with a solvent, filtering, and drying a filter cake, and the solvent for the slurrying preferably comprises methyl tert-butyl ether or diethyl ether.

In some embodiments of the preparation of compound (V) in the present invention, when P is selected from tertbutoxycarbonyl, the molar ratio of the reducing agent to the compound (VI) is ≤ 5 : 1, preferably 2 : 1-4 : 1.

In some embodiments of the preparation of compound (V) in the present invention, when P is selected from tertbutoxycarbonyl, the molar ratio of the acidic reagent to the compound (VI) is ≤ 5 : 1, preferably 2 : 1-4 : 1.

In some embodiments of the preparation of compound (V) in the present invention, when P is selected from tertbutoxycarbonyl, the molar ratio of the compound 1a to the compound (VI) is ≤ 5 : 1, preferably 2 : 1-4 : 1.

In some embodiments of the preparation of compound (VII) in the present invention, when P is selected from

tertbutoxycarbonyl, the molar ratio of the reducing agent to the compound (VIII) is ≤ 5 : 1, preferably 2 : 1-4 : 1.

In some embodiments of the preparation of compound (VII) in the present invention, when P is selected from tertbutoxycarbonyl, the molar ratio of the acidic reagent to the compound (VIII) is ≤ 5 : 1, preferably 2 : 1-4 : 1.

In some embodiments of the preparation of compound (VII) in the present invention, when P is selected from tertbutoxycarbonyl, the molar ratio of the compound 1a to the compound (VIII) is ≤ 5 : 1, preferably 2 : 1-4 : 1.

The methods for preparing compound (VII), compound (V), compound (IV) or compound (VI-1) described in the present invention have the following advantages: the method has mild reaction conditions, does not involve a high-temperature and high-pressure reaction, and has low-toxicity or non-toxicity raw materials, simple operation, less by-products, a high product purity and convenient work-up.

The present invention relates to a method for preparing compound (I), the method comprising the following steps:

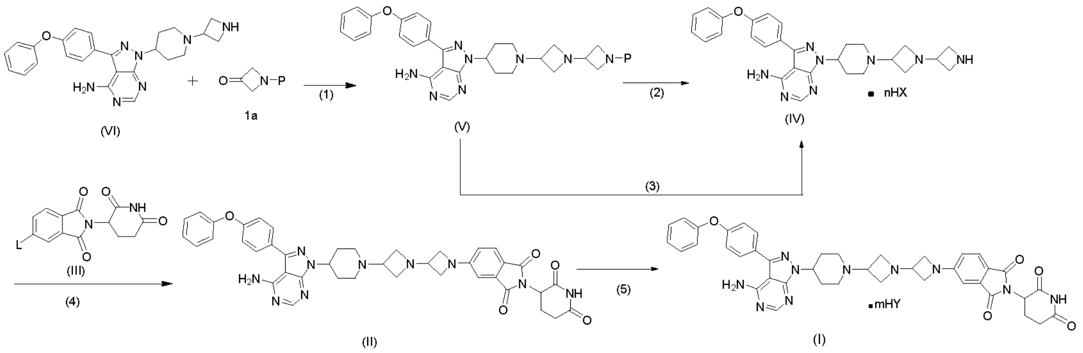


wherein L is selected from a trifluoromethanesulphonate group, F, Cl, Br, I,

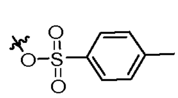, 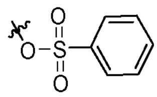, or 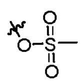;

HX is selected from acetic acid, hydrochloric acid, sulphuric acid, hydrobromic acid, hydroiodic acid or trifluoroacetic acid;
n is selected from 0, 1, 1.5, 2, 3 or 4;
HY is selected from a pharmaceutically acceptable salt, preferably fumarate, formate, acetate, butanedioate, hydrochloride, sulphate, tartrate, p-methylbenzoate, methanesulphonate, malate, maleate and succinate;
m is selected from 0.5, 1, 1.5, 2 or 3;
in step (1), compound (VI) is reacted with 1a in the presence of an acidic reagent and a reducing agent, optionally with the addition of a desiccant, and then subjected to a work-up to obtain compound (V);
in step (2), the compound (V) is reacted in the presence of an acidic reagent HX to obtain compound (IV), wherein the HX is selected from acetic acid, sulphuric acid, hydrobromic acid, hydroiodic acid or trifluoroacetic acid;
in step (3), the compound (V) is reacted in the presence of hydrochloric acid and a polar protic solvent to obtain the compound (IV), wherein the HX is HCl;
in step (4), the compound (IV) is reacted with compound (III) in the presence of an alkaline reagent to obtain compound (II); and
in step (5), the compound (II) is reacted with HY to prepare the compound (I).

In some embodiments of the preparation of compound (I) in the present invention, in step (2), a reaction is performed in the presence of an acidic reagent HX and a solvent to obtain compound (IV), and after the reaction is completed, an alkaline reagent is optionally added to neutralize the acid in the system so as to obtain the compound (IV) in the form of a free base (i.e., in this case n = 0).

In some embodiments of the preparation of compound (I) in the present invention, in step (3), a reaction is performed in the presence of hydrochloric acid and a polar protic solvent to obtain compound (IV), wherein the HX is HCl, and after the reaction is completed, an alkaline reagent is optionally added to neutralize the acid in the system so as to obtain the compound (IV) in the form of a free base (i.e., in this case n = 0).

In some embodiments of the preparation of compound (I) in the present invention,

in step (1), the acidic reagent is selected from one or more of hydrochloric acid, acetic acid, formic acid, propionic

acid, butyric acid, sulphuric acid, hydrobromic acid, hydroiodic acid or trifluoroacetic acid, the reducing agent is selected from a boron reducing agent, preferably one or more of sodium borohydride, sodium triacetoxyborohydride, sodium triethylborohydride, sodium cyanoborohydride, potassium borohydride or lithium borohydride, and the desiccant is selected from one or more of anhydrous sodium sulphate, anhydrous magnesium sulphate, anhydrous calcium sulphate or a molecular sieve;

and the alkaline reagent in step (4) is selected from an organic amine reagent, preferably one or more of triethylamine, diethylamine or N,N-diisopropylethylamine.

In some embodiments of the preparation of compound (I) in the present invention,

in step (1), the acidic reagent comprises one or more of hydrochloric acid, acetic acid, formic acid, propionic acid, butyric acid, sulphuric acid, hydrobromic acid, hydroiodic acid or trifluoroacetic acid, the reducing agent comprises a boron reducing agent, preferably one or more of sodium borohydride, sodium triacetoxyborohydride, sodium triethylborohydride, sodium cyanoborohydride, potassium borohydride or lithium borohydride, and the desiccant comprises one or more of anhydrous sodium sulphate, anhydrous magnesium sulphate, anhydrous calcium sulphate or a molecular sieve;

and the alkaline reagent in step (4) comprises an organic amine reagent, preferably one or more of triethylamine, diethylamine or N,N-diisopropylethylamine.

In some embodiments of the preparation of compound (I) in the present invention,

step (1) involves a solvent selected from one or more of 1,2-dichloroethane, chloroform or dichloromethane;

step (2) involves a solvent selected from a polar aprotic solvent or a polar protic solvent, preferably one or more of dichloromethane, 1,2-dichloroethane, chloroform, carbon tetrachloride, tetrahydrofuran, methanol, ethanol and water;

in step (3), the solvent is selected from one or more of methanol, ethanol and water, a polar aprotic solvent is optionally further added to the reaction, and the polar aprotic solvent is preferably one or more of dichloromethane, 1,2-dichloroethane, chloroform, carbon tetrachloride or tetrahydrofuran;

step (4) involves a solvent selected from a polar aprotic solvent, preferably one or more of N,N-dimethylformamide, N,N-diethylformamide, N,N-dimethylacetamide, dimethylsulphoxide or N-methyl-2-pyrrolidone;

and step (5) involves a solvent selected from one or more of dichloromethane, 1,2-dichloroethane, ethyl acetate, acetone, methanol, ethanol, ethylene glycol, polyethylene glycol, isopropanol, diethyl ether, tetrahydrofuran and water.

In some embodiments of the preparation of compound (I) in the present invention,

step (1) involves a solvent, which comprises one or more of 1,2-dichloroethane, chloroform or dichloromethane;

step (2) involves a solvent selected from a polar aprotic solvent or a polar protic solvent, preferably one or more of dichloromethane, 1,2-dichloroethane, chloroform, carbon tetrachloride, tetrahydrofuran, methanol, ethanol and water;

the solvent in step (3) comprises one or more of methanol, ethanol and water, a polar aprotic solvent is optionally further added to the reaction, and the polar aprotic solvent comprises one or more of dichloromethane, 1,2-dichloroethane, chloroform, carbon tetrachloride or tetrahydrofuran;

step (4) involves a solvent, which comprises a polar aprotic solvent, preferably one or more of N,N-dimethylformamide, N,N-diethylformamide, N,N-dimethylacetamide, dimethylsulphoxide or N-methyl-2-pyrrolidone;

and step (5) involves a solvent, which comprises one or more of dichloromethane, 1,2-dichloroethane, ethyl acetate, acetone, methanol, ethanol, ethylene glycol, polyethylene glycol, isopropanol, diethyl ether, tetrahydrofuran and water.

In some embodiments of the preparation of compound (I) in the present invention,

the reaction in step (1) is performed at a temperature of 0°C-40°C, preferably 20°C-40°C;

the reaction in step (2) is performed at a temperature of 0°C-40°C, preferably 20°C-40°C;

the reaction in step (3) is performed at a temperature of 0°C-40°C, preferably 20°C-40°C;

the reaction in step (4) is performed at a temperature of 30°C-120°C, preferably 60°C-110°C, more preferably 80°C-100°C;

and the reaction in step (5) is performed at a temperature of 0°C-40°C, preferably 10°C-30°C.

The present invention relates to a compound as represented below:

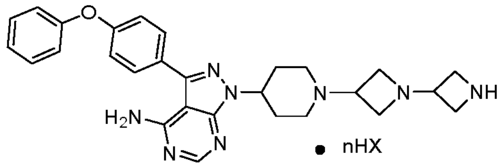

(IV)

wherein HX is selected from acetic acid, sulphuric acid, hydrobromic acid, hydroiodic acid or trifluoroacetic acid; and n is selected from 1, 1.5, 2, 3 or 4.

The compound of formula (IV) involved in the present invention has a better stability and is suitable for long-term storage. The preparation of compound (II), which uses the compound of formula (IV) as an intermediate, has the following advantages: the reaction has a high yield and produces less by-products and impurities; and the purification process is simple.

The present invention relates to a method for refining a compound as represented by formula (I), the method comprising mixing the compound as represented by formula (I) with a solvent comprising methanol, ethanol, isopropanol, ethyl acetate, acetone, methyl tert-butyl ether, diethyl ether or water, and crystallizing or/and slurrying the resulting mixture,

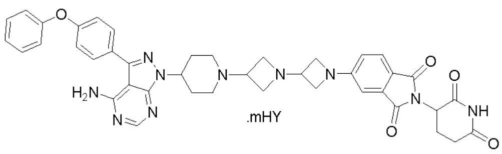

(I)

wherein HY is selected from a pharmaceutically acceptable salt, preferably fumaric acid, formic acid, acetic acid, butanedioic acid, hydrochloric acid, sulphuric acid, tartaric acid, p-methylbenzoic acid, methanesulphonic acid, malic acid, maleic acid and succinic acid; and
m is selected from 0.5, 1, 1.5, 2 or 3.

The present invention relates to a method for refining a compound as represented by formula (I), the method comprising mixing the compound as represented by formula (I) with one or more solvents selected from methanol, ethanol, isopropanol, ethyl acetate, acetone, methyl tert-butyl ether, diethyl ether or water, and crystallizing or/and slurrying the resulting mixture.

The present invention relates to a method for refining a compound as represented by formula (II), the method comprising mixing the compound as represented by formula (II) with a solvent comprising methanol, ethanol, isopropanol, ethyl acetate, acetone, methyl tert-butyl ether, diethyl ether or water, and crystallizing or/and slurrying the resulting mixture,

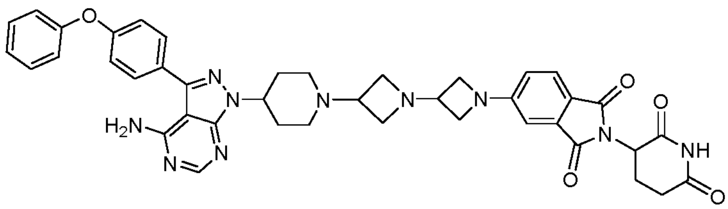

(II)

.

The present invention relates to a method for refining a compound as represented by formula (II), the method comprising mixing the compound as represented by formula (II) with one or more solvents selected from methanol, ethanol, isopropanol, ethyl acetate, acetone, methyl tert-butyl ether, diethyl ether or water, and crystallizing or/and slurrying the resulting mixture.

The present invention relates to a method for refining a compound as represented by formula (IV), the method comprising mixing the compound as represented by formula (IV) with a solvent comprising methanol, ethanol, isopropanol, ethyl acetate, acetone, methyl tert-butyl ether, diethyl ether or water, and crystallizing or/and slurrying the resulting mixture,

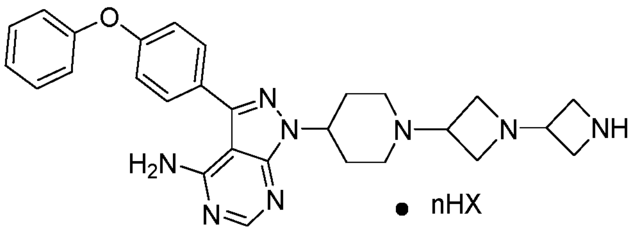

• nHX

(IV)

wherein HX is selected from hydrochloric acid, acetic acid, sulphuric acid, hydrobromic acid, hydroiodic acid or trifluoroacetic acid; and
n is selected from 0, 1, 1.5, 2, 3 or 4.

The present invention relates to a method for refining a compound as represented by formula (IV), the method comprising mixing the compound as represented by formula (IV) with one or more solvents selected from methanol, ethanol, isopropanol, ethyl acetate, acetone, methyl tert-butyl ether, diethyl ether or water, and crystallizing or/and slurrying the resulting mixture.

The present invention relates to a method for refining a compound as represented by formula (V) or (VII), the method comprising mixing the compound as represented by formula (V) or (VII) with a solvent comprising methanol, ethanol, isopropanol, ethyl acetate, acetone, methyl tert-butyl ether, diethyl ether or water, and crystallizing or/and slurrying the resulting mixture,

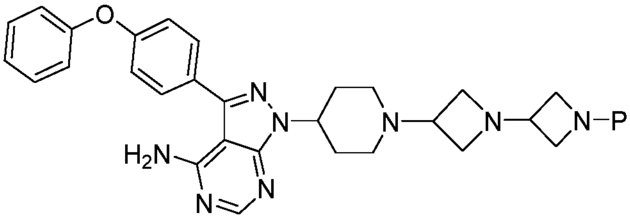

(V)

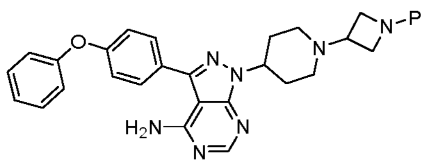

(VII)

wherein P is selected from an amino protecting group, preferably tertbutoxycarbonyl, benzyloxycarbonyl, methoxycarbonyl, ethoxycarbonyl, p-toluenesulphonyl, trifluoroacetyl, triphenylmethyl and p-methoxybenzyl.

The present invention relates to a method for refining a compound as represented by formula (V) or (VII), the method comprising mixing the compound as represented by formula (V) or (VII) with one or more solvents selected from methanol, ethanol, isopropanol, ethyl acetate, acetone, methyl tert-butyl ether, diethyl ether or water, and crystallizing or/and slurrying the resulting mixture.

Unless stated to the contrary, the terms used in the description and claims have the following meanings.

The extraction method used in the work-up of the reaction in the present invention is a conventional method in the art; the extraction solvent can be selected according to the solubility of a product and the solubility of an organic solvent in water; and common extraction solvents include but are not limited to one of or mixed solvents of two or more of dichloromethane, chloroform, ethyl acetate, methyl acetate, isopropyl acetate, diethyl ether, isopropyl ether, methyl tert-butyl ether, methanol and ethanol. The number of extractions can be appropriately increased or decreased according to the amount of the remaining products in an aqueous phase. Optionally, the organic phase that has been extracted may be further subjected to conventional washing or/and drying treatment in the art.

The carbon, hydrogen, oxygen, sulphur, nitrogen or halogen elements involved in the groups and compounds of the present invention all comprise their isotope forms, and the carbon, hydrogen, oxygen, sulphur, or nitrogen elements involved in the groups and compounds of the present invention are optionally further substituted with 1 to 5 of their corresponding isotopes, wherein the isotopes of carbon comprise $^{12}C$, $^{13}C$ and $^{14}C$, the isotopes of hydrogen comprise protium (H), deuterium (D, also known as heavy hydrogen), and tritium (T, also known as superheavy hydrogen), the

isotopes of oxygen comprise $^{16}O$, $^{17}O$ and $^{18}O$, the isotopes of sulphur comprise $^{32}S$, $^{33}S$, $^{34}S$ and $^{36}S$, the isotopes of nitrogen comprise $^{14}N$ and $^{15}N$, the isotopes of fluorine comprise $^{19}F$, the isotopes of chlorine comprise $^{35}Cl$ and $^{37}Cl$, and the isotopes of bromine comprise $^{79}Br$ and $^{81}Br$.

The term "alcohol solvent" refers to a solvent containing hydroxyl in the molecular structure, and non-limiting examples of alcohol solvents include ethylene glycol, methanol, ethanol, n-propanol, isopropanol, n-butanol, n-pentanol, sec-pentanol, 3-pentanol, isopentanol, tertiary pentanol, n-hexanol, cyclohexanol, etc.

The term "ether solvent" refers to a solvent containing an ether bond in the molecular structure, and non-limiting examples of ether solvents include tetrahydrofuran, 2-methyl tetrahydrofuran, diethyl ether, 1,4-dioxane, methyl tert-butyl ether, ethylene glycol dimethyl ether, diisopropyl ether, ethylbutyl ether, dibutyl ether, dipentyl ether, diethylene glycol dimethyl ether, triethylene glycol dimethyl ether, anisole, etc.

The term "aromatic hydrocarbon solvent" refers to a solvent containing an aryl ring having 0-3 heteroatoms (the heteroatoms are selected from O, S or N) in the molecular structure, and non-limiting examples of aromatic hydrocarbon solvents include benzene, pyridine, toluene, ethyl benzene, xylene, chlorobenzene, o-dichlorobenzene, etc.

The term "halogenated alkane solvent" refers to an alkane solvent containing halogens (fluorine, chlorine, bromine, iodine) in the molecular structure, and non-limiting examples of halogenated alkane solvents include dichloromethane, 1,2-dichloroethane, chloroform, trichloroethane, carbon tetrachloride, pentachlorohexane, 1-chlorobutane, tribromomethane, etc.

The term "alkane solvent" refers to a solvent containing only alkane in the molecular structure, and non-limiting examples of alkane solvents include n-hexane, n-heptane, n-octane, n-pentane, cyclohexane, cycloheptane, etc.

The term "ester solvent" refers to a solvent containing a carboxylic ester in the molecular structure, and non-limiting examples of ester solvents include ethyl acetate, isopropyl acetate, glyceryl triacetate, ethyl acetoacetate, isoamyl acetate, isopropyl acetate, n-butyl acetate, n-propyl acetate, n-amyl acetate, methyl acetate, sec-butyl acetate, butyl formate, propyl formate, n-pentyl formate, diethyl carbonate, etc.

The term "ketone solvent" refers to a solvent containing ketocarbonyl in the molecular structure, and non-limiting examples of ketone solvents include acetone, butanone, acetophenone, methyl isobutyl ketone, 2,6-dimethyl-2,5-heptadiene-4-one, 3,5,5-trimethyl-2-cyclohexenone, mesityl oxide, etc.

The term "nitrile solvent" refers to a solvent containing cyano in the molecular structure, and non-limiting examples of nitrile solvents include acetonitrile, propionitrile, butyronitrile, phenylacetonitrile, etc.

The term "amide solvent" refers to a solvent containing amide in the molecular structure, and non-limiting examples of amide solvents include N,N-dimethylformamide, N,N-dimethylacetamide, N,N-diethylacetamide, hexamethylphosphoramide, N-methyl pyrrolidone, etc.

The term "polar aprotic solvent" refers to a solvent that does not contain a hydrogen atom directly connected to an electronegative atom and has no hydrogen bonding ability. Non-limiting examples of polar aprotic solvents include acetone, dimethyl sulphoxide, HMF (hydroxymethylfurfural), crown ether, acetonitrile, N,N-dimethylformamide, N,N-diethylformamide, N,N-dimethylacetamide, dimethylsulphoxide, or N-methyl-2-pyrrolidone, etc.

The term "polar protic solvent" refers to solvents that have a hydrogen bonding ability (because they contain at least one hydrogen atom directly connected to an electronegative atom (such as O-H or N-H bond)), and non-limiting examples of polar protic solvents include methanol, water, ethanol, ammonia, acetic acid, etc.

"Optionally" or "as an alternative" means that events or circumstances subsequently described may but not necessarily occur, and the description includes the occasions where the events or circumstances occur or do not occur.

During the reaction of the present invention, the reaction process is tracked by HPLC, HNMR or thin layer chromatography so as to judge whether the reaction is completed.

In the present invention, the internal temperature indicates the temperature of the reaction system.

## Detailed Description of Embodiments

The implementation process and beneficial effects of the present invention are described in detail below through specific examples, which are intended to help readers better understand the essence and characteristics of the present invention and are not intended to limit the scope of implementation of the present invention.

The structures of the compounds are determined by nuclear magnetic resonance (NMR) or (and) mass spectrometry (MS). The NMR shift (δ) is given in the unit of $10^{-6}$ (ppm). NMR is measured with (BrukerAvance III 400 and BrukerAvance 300) NMR instrument, and the solvent for determination is deuterated dimethyl sulphoxide (DMSO-$d_6$), deuterated chloroform ($CDCl_3$), deuterated methanol ($CD_3OD$), and deuterated acetonitrile ($CD_3CN$), and the internal standard is tetramethylsilane (TMS); MS is measured with the mass spectrometer Agilent 6120 Quadrupole MS.

Example 1

(1) Synthesis of compound 1B (i.e., tert-butyl 3-(4-(4-amino-3-(4-phenoxyphenyl)-1H-pyrazolo[3,4-d]pyrimidin-1-yl)piperidin-1-yl)azetidine-1-carboxylate)

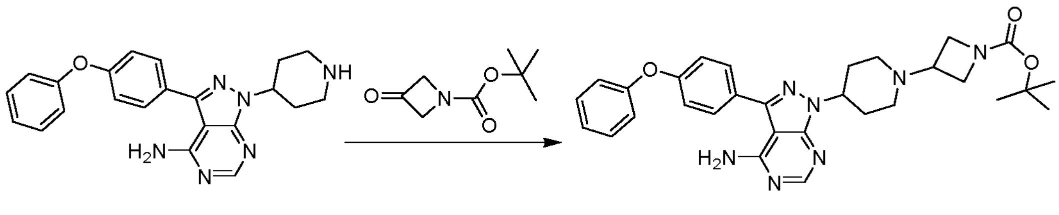

1A 1B

To a 2 L three-necked flask equipped with a thermometer and provided with mechanical stirring, dichloromethane (350 g) was added, and then at around 25°C, compound 1A (69.9 g, 0.181 mol), tert-butyl 3-oxoazetidine-1-carboxylate (62.3 g, 0.364 mol), acetic acid (25.0 g, 0.416 mol), and anhydrous sodium sulphate (86.2 g, 0.607 mol) were successively added with stirring. After the mixture was stirred for 1 h, sodium triacetoxyborohydride (79.9 g, 0.377 mol) was slowly added in portions at 15°C to 20°C. Upon completion of addition, the mixture was reacted at 25°C to 35°C for about 3 to 5 hours.

After the reaction was completed, the reaction solution was filtered, and the resulting filtrate was neutralized with 15% sodium hydroxide aqueous solution until the aqueous phase reached about pH 10. After liquid separation was performed, the aqueous phase was extracted, and the combined organic phase was washed with water, dried, and concentrated under reduced pressure at around 40°C to obtain a yellow viscous substance, which was crystallized with methyl tert-butyl ether to obtain a white solid. The white solid was dried under reduced pressure at 40°C to 50°C to obtain compound 1B, i.e., tert-butyl 3-(4-(4-amino-3-(4-phenoxyphenyl)-1H-pyrazolo[3,4-d]pyrimidin-1-yl)piperidin-1-yl)azetidine-1-carboxylate (94.4 g, yield: 96.2%, purity: 98.9%).

$^1$HNMR (DMSO-d6): δ 8.25 (s, 1H), 7.67 (d, 2H), 7.45 (t, 2H), 7.21-7.11 (br, m, 7H), 4.70 (s, 1H), 3.86-3.69 (br, 4H), 3.13-3.07 (m, 1H), 2.96-2.89 (m, 2H), 2.05-1.99 (m, 2H), 2.26-2.19 (m, 2H), 1.93-1.90 (m, 2H), 1.40 (s, 9H).

(+)ESI-MS: 542.2 [M+1].

(2) Synthesis of compound 1C (i.e., 1-(1-(azetidin-3-yl)piperidin-4-yl)-3-(4-phenoxyphenyl)-1H-pyrazolo[3,4-d]pyrimidin-4-amine)

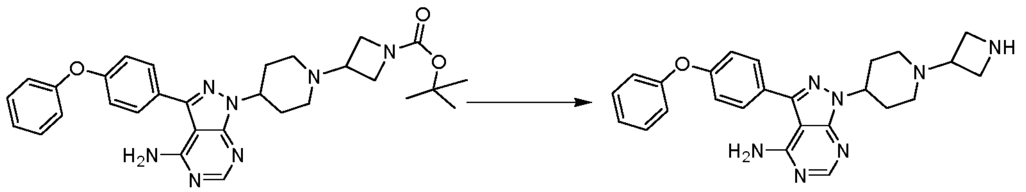

1B 1C

To a 2 L three-necked flask equipped with a thermometer and provided with mechanical stirring, dichloromethane (539 g) and compound 1B (107.8 g, 0.200 mol) were successively added with stirring. After the mixture was dissolved to yield a clear solution, trifluoroacetic acid (318.0 g, 2.789 mol) was added whilst the temperature was controlled at 10°C to 15°C. The resulting mixture was reacted at 25°C to 30°C for about 3 hours.

After the reaction was completed, the reaction solution was concentrated under reduced pressure at 30°C to 45°C to obtain a light yellow viscous substance. Then the light yellow viscous substance was dissolved with dichloromethane (500 g) and the resulting solution was neutralized with 20% sodium hydroxide aqueous solution until the aqueous phase reached about pH 11 whilst the temperature was controlled at 0°C to 15°C.

After liquid separation was performed, the aqueous phase was extracted, and the combined organic phase was washed with water, dried, and concentrated under reduced pressure at 30°C to 45°C. Finally, the residue was crystallized with methyl tert-butyl ether to obtain a white solid, which was dried under reduced pressure at 40°C to 50°C

to obtain compound 1C, i.e., 1-(1-(azetidin-3-yl)piperidin-4-yl)-3-(4-phenoxyphenyl)-1H-pyrazolo[3,4-d]pyrimidin-4-amine (76.3 g, yield: 86.9%, purity: 98.4%).

$^1$HNMR (DMSO-d6): δ 1.9 (m, 2H), 2.0 (m, 2H), 2.2 (m, 2H), 2.8 (m, 2H), 2.9 (m, 1H), 3.0 (m, 2H), 3.5 (m, 2H), 4.7 (m, 1H), 7.1-7.2 (m, 5H), 7.4-7.5 (t, 2H), 7.6-7.7 (d, 2H), 8.2 (s, 1H).

$^1$HNMR (DMSO-d6): δ 8.25 (s, 1H), 7.67 (d, 2H), 7.45-7.42 (m, 2H), 7.21-7.11 (m, 5H), 6.93 (br, 2H), 4.70-4.64 (m, 1H), 3.97 (br, 1H), 3.53-3.52 (m, 2H), 3.17-3.05 (m, 2H), 2.89-2.84 (m, 3H), 2.24-2.16 (m, 2H), 2.00-1.90 (m, 4H).

(+)ESI-MS:442.2[M+1].

(3) Synthesis of compound 1E (tert-butyl 3-(4-(4-amino-3-(4-phenoxyphenyl)-1H-pyrazolo[3,4-d]pyrimidin-1-yl)piperidin-1-yl)-[1,3']-biazetidine-1'-carboxylate)

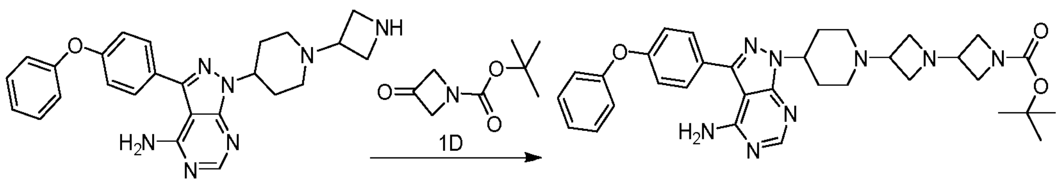


1C 1E

To a 2 L three-necked flask equipped with a thermometer and provided with mechanical stirring, dichloromethane (600 g) was added, and then at around 25°C, 1C (99.8 g, 0.226 mol), tert-butyl 3-oxoazetidine-1-carboxylate (1D) (78.1 g, 0.456 mol), acetic acid (32.0 g, 0.533 mol), and anhydrous sodium sulphate (86.9 g, 0.612 mol) were successively added with stirring. After the mixture was stirred for 1 h, sodium triacetoxyborohydride (96.0 g, 0.453 mol) was slowly added in portions. Upon completion of addition, the mixture was reacted at 25°C to 35°C for about 3 to 5 hours.

After the reaction was completed, the reaction solution was filtered, and the resulting filtrate was neutralized with 15% sodium hydroxide aqueous solution until the aqueous phase reached about pH 11. After liquid separation was performed, the aqueous phase was extracted, and the combined organic phase was washed with water, dried, and concentrated under reduced pressure to obtain a yellow viscous substance, which was crystallized with methyl tert-butyl ether to obtain a white solid. The white solid was dried under reduced pressure at 40°C to 50°C to obtain compound 1E, i.e., tert-butyl-1-carboxylate-3-(4-(4-amino-3-(4-phenoxyphenyl)-1H-pyrazolo[3,4-d]pyrimidin-1-yl)piperidin-1-yl)-[1,3'-biazetidine (100.3 g, yield: 74.2%, purity: 89.1%).

$^1$HNMR (DMSO-d6): δ 8.23 (s, 1H), 7.67-7.65 (m, 2H), 7.46 - 7.42 (m, 2H), 7.21-7.12 (m, 5H), 6.93 (br, 2H), 4.69 - 4.63 (m, 1H), 3.82 (m, 4H), 3.39 - 3.35 (m, 3H), 2.97 - 2.92 (m, 2H), 2.88 - 2.82 (m, 2H), 2.02 - 1.96 ( m, 2H), 2.23 - 2.15 (m, 2H), 1.91- 1.89 ( m, 2H), 1.37 (s, 9H).

(+)ESI-MS:597.3[M+1].

(4) Synthesis of compound 1F (i.e., 1-(1-([1,3'-diazepane]-3-yl)piperidin-4-yl)-3-(4-phenoxyphenyl)-1H-pyrazoline[3,4-d]pyrimidin-4-amine tetratrifluoroacetate)

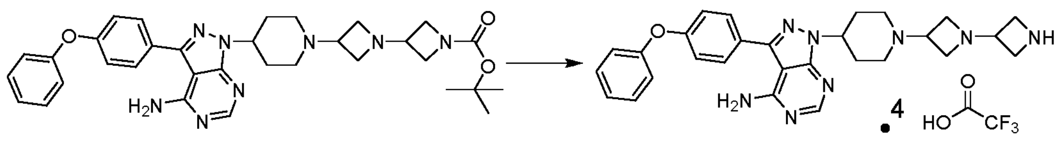

1E 1F

To a 2 L three-necked flask equipped with a thermometer and provided with mechanical stirring, dichloromethane (350 g) and compound 1E (69.8 g, 0.117 mol) were successively added with stirring. After the mixture was dissolved to yield a clear solution, trifluoroacetic acid (209.8 g, 1.84 mol) was added whilst the temperature was controlled at 15°C to 35°C. The resulting mixture was reacted at 25°C to 30°C for about 3 hours.

After the reaction was completed, the reaction solution was concentrated under reduced pressure at 30°C to 45°C to obtain a light yellow viscous substance, which was then slurried 3 times with methyl tert-butyl ether to obtain a

white solid. The white solid was dried under reduced pressure at 40°C to 50°C to obtain compound 1F, i.e., 1-(1-([1,3'-diazepane]-3-yl)piperidin-4-yl)-3-(4-phenoxyphenyl)-1H-pyrazoline[3,4-d]pyrimidin-4-amine tetratrifluoroacetate (105.2 g, yield: 94.4%, purity: 88.9%).

$^{1}$HNMR (DMSO-d6): δ 8.23 (s, 1H), 7.69 -7.65 (m, 2H), 7.46-7.41 (m, 2H), 7.21-7.11 (m, 5H), 6.89 (br, 2H), 4.70 - 4.62 (m, 1H), 3.44 - 3.30 (m, 7H), 2.98-2.81 (m, 1H), 2.88 - 2.82 (m, 2H), 2.24 - 2.14 (m, 2H), 2.00 - 1.94 (m, 6H), 1.91 - 1.88 (m, 2H).

(+)ESI-MS:497.3[M+1].

The number of trifluoroacetic acids in compound 1F was determined by HPLC, and the results were shown as follows:

| Determination of number of trifluoroacetic acids in compound | | | | | | | | | | |
|---|---|---|---|---|---|---|---|---|---|---|
| | Sample weight/mg | Dilution fold | Concentration (mg) | Peak area | F | Average of F | | | | |
| Reference solution 1 | 122.46 | 5000 | 0.024 | 151632 | 1.615E-07 | 1.615E-07 | | | | |
| Reference solution 2 | 125.68 | | 0.025 | 155533 | 1.615E-07 | | | | | |
| Sample to be tested | | | | Trifluoroacetic acid | | | | | | Number of trifluoroacetic acids/free bases |
| | Sample weight /mg | Dilution fold | Peak | Content/ % | Average of content /% | area Amount/mg | Molecular weight | Mole number mol | Mean | |
| | 12.52 | 250-fold | 144967 | 46.38 | 46.55 | 5.853 | 114.02 | 0.0513 | 0.0257 | 4 |
| | | | | 46.71 | | 0.000 | | 0.0000 | | |
| | | | | Fee base | | | | | | |
| | 12.76 | | 147609 | Content/ % | Average of content/ % | Amount/mg | Molecular weight | Mole number mol | Mean | |
| | | | | 53.62 | 53.46 | 6.767 | 496.27 | 0.0136 | 0.0068 | |
| | | | | 53.29 | | 0.000 | | 0.0000 | | |

(5) Synthesis of compound 1 (i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2, 6-dioxo-3-piperidyl)isoindoline-1,3 -dione)

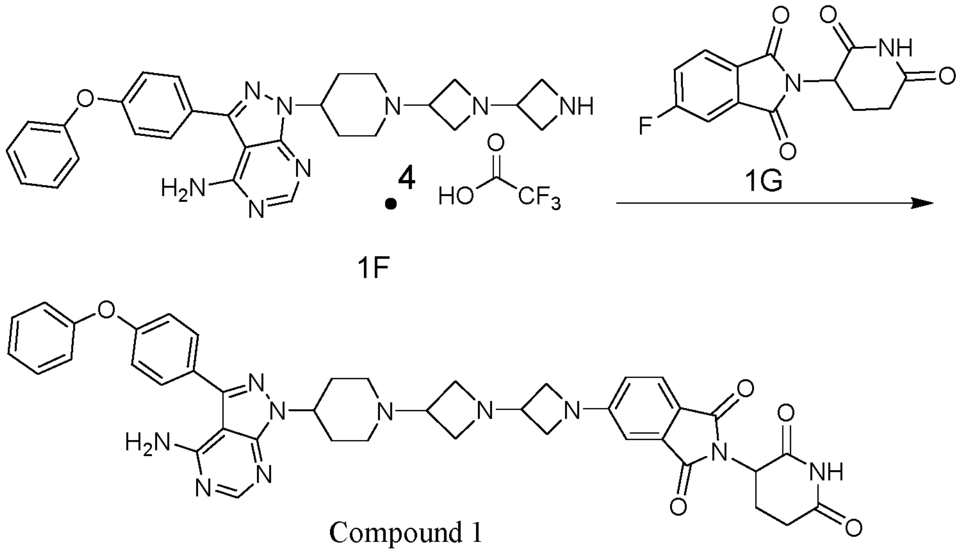

1F

Compound 1

At around 20°C, to a 2 L three-necked flask equipped with a thermometer and provided with mechanical stirring, dimethylsulphoxide (90 g), compound 1F (90.8 g, 0.095 mol), and N,N-diisopropylethylamine (82.0 g, 0.63 mol) were successively added with stirring to form a solution. The solution was then added to a 60°C to 70°C solution containing dimethylsulphoxide (400 g) and compound 1G (i.e., 2-(2,6-dioxopiperidin-3-yl)-5-fluoroisoindole-1,3-dione) (36.0 g, 0.13 mol), and the resulting mixture was reacted at 85°C to 95°C for 6 to 8 hours.

After the reaction was completed, the reaction solution was cooled to 15°C to 25°C. Liquid separation was performed to remove the upper layer, which contains the vast majority of N,N-diisopropylethylamine. The reaction solution in the lower layer was poured into water (2.7 L) for crystallization. After filtering, the resulting mixture was slurried with anhydrous ethanol (0.54 Kg) for 1 hour and then filtered to obtain compound 1 as a yellow solid, i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione (56.2 g, yield: 67.9%, purity: 85.1%).

$^1$HNMR (DMSO-d6): $\delta$ 11.06 (s, 1H), 8.24 (s, 1H), 7.68-7.62 (m, 3H), 7.46-7.41 (m, 2H), 7.21-7.11 (m, 5H), 6.79 (d, 1H), 6.65 (dd, 1H), 5.06 (dd, 1H), 4.69-4.64 (m, 1H), 4.04 (t, 2H), 3.81 (dd, 2H), 3.66-3.63 (m, 1H), 3.42 (s, 2H), 2.99-2.92 (m, 3H), 2.88-2.84 (m, 3H), 2.61-2.54 (m, 1H), 2.51 (d, 1H), 2.24-2.16 (m, 2H), 2.00 (dd, 3H), 1.90 (d, 2H).

(+)ESI-MS:753.3 [M+1].

(6) Synthesis of compound 1-1 (i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione dimaleate)

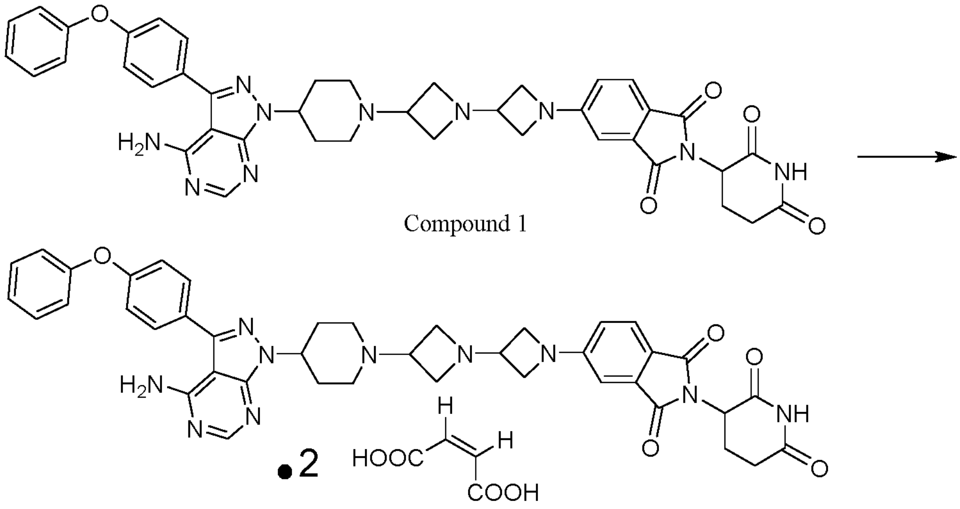

Compound 1

Compound 1-1

At around 20°C, to a 2 L three-necked flask equipped with a thermometer and provided with mechanical stirring, dichloromethane (747.5 g), methanol (18.5 g), and compound 1 (56.2 g, 0.075 mol) were successively added with stirring. Then a solution containing methanol (22.5 g) and maleic acid (17.4 g, 0.15 mol) was added at 10°C to 20°C. Subsequently, the reaction solution was cooled to around 0°C for crystallization for about 4 hours.

After filtering, the resulting mixture was dried under reduced pressure at 40°C to 50°C to obtain a crude, which was further refined and purified to obtain compound 1-1 as a yellow solid, i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione dimaleate (41.9 g, yield: 56.7%, purity: 99.1%).

$^1$HNMR (DMSO-d6): δ 11.09 (s, 1H), 8.27 (s, 1H), 7.70 ~ 7.66 (m, 3H), 7.47 ~ 7.43 (m, 2H), 7.22-7.13 (m, 5H), 6.87 (d, 1H), 6.74 ~ 6.71 (m, 1H), 6.16 (s, 4H), 5.10 ~ 5.05 (m, 1H), 4.97 ~ 4.92 (m, 1H), 4.20 ~ 4.17 (m, 2H), 4.05 (br, 1H), 3.99 ~ 3.97 (m, 2H), 3.87 (br, 2H), 3.70 (br, 3H), 3.32 ~ 3.30 (m, 2H), 2.94 ~ 2.84 (m, 1H), 2.87 (br, 2H), 2.62 ~ 2.54 (m, 2H), 2.44 ~ 2.35 (m, 2H), 2.14-2.11 (m, 2H), 2.04-2.01 (m, 1H).

(+)ESI-MS:753.3 [M+1].

Example 2

(1) Synthesis of compound 1B (i.e., tert-butyl 3-(4-(4-amino-3-(4-phenoxyphenyl)-1H-pyrazolo[3,4-d]pyrimidin-1-yl)piperidin-1-yl)azetidine-1-carboxylate)

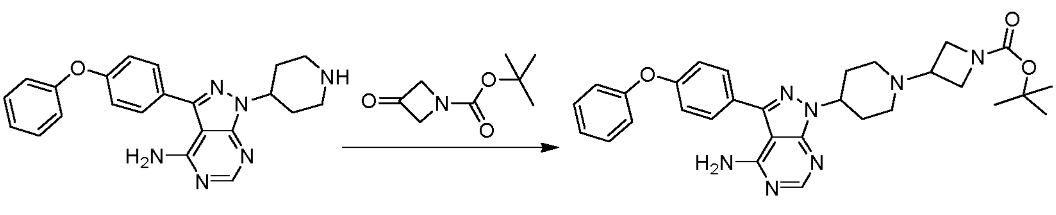

**1A** **1B**

To a 2 L three-necked flask equipped with a thermometer and provided with mechanical stirring, dichloromethane (450 g) was added, and then at around 25°C, compound 1A (100.1 g, 0.259 mol), tert-butyl 3-oxoazetidine-1-carboxylate (96.6 g, 0.564 mol), formic acid (23.8 g, 0.518 mol), and anhydrous sodium sulphate (80.9 g, 0.570 mol) were successively added with stirring. After the mixture was stirred for 1 h, sodium triacetoxyborohydride (120.8, 0.570 mol) was slowly added in portions at 15°C to 20°C. Upon completion of addition, the mixture was reacted at 25°C to 35°C for about 3 to 5 hours.

After the reaction was completed, the reaction solution was filtered, and the resulting filtrate was neutralized with 15% sodium hydroxide aqueous solution until the aqueous phase reached about pH 10. After liquid separation was performed, the aqueous phase was extracted, and the combined organic phase was washed with water, dried, and

concentrated under reduced pressure at around 40°C to obtain a yellow viscous substance, which was crystallized with methyl tert-butyl ether to obtain a white solid. The white solid was dried under reduced pressure at 40°C to 50°C to obtain compound 1B, i.e., tert-butyl 3-(4-(4-amino-3-(4-phenoxyphenyl)-1H-pyrazolo[3,4-d]pyrimidin-1-yl)piperidin-1-yl)azetidine-1-carboxylate (77.6 g, yield: 95.2%, purity: 99.2%).

(2) Synthesis of compound 1C (i.e., 1-(1-(azetidin-3-yl)piperidin-4-yl)-3-(4-phenoxyphenyl)-1H-pyrazolo[3,4-d]pyrimidin-4-amine)

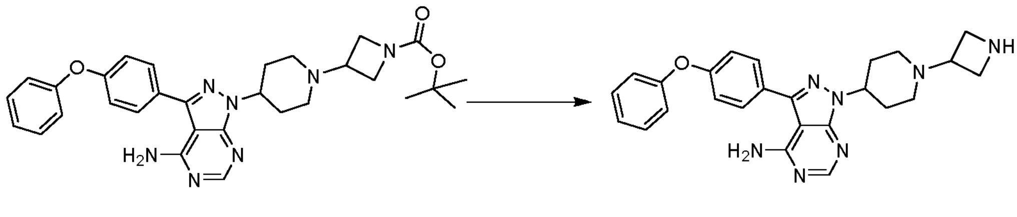

1B 1C

To a 2 L three-necked flask equipped with a thermometer and provided with mechanical stirring, dichloromethane (539 g) and compound 1B (107.8 g, 0.200 mol) were successively added with stirring. After the mixture was dissolved to yield a clear solution, trifluoroacetic acid (318.0 g, 2.789 mol) was added whilst the temperature was controlled at 10°C to 15°C. The resulting mixture was reacted at 25°C to 30°C for about 3 hours.

After the reaction was completed, the reaction solution was concentrated under reduced pressure at 30°C to 45°C to obtain a light yellow viscous substance. Then the light yellow viscous substance was dissolved with dichloromethane (500 g) and the resulting solution was neutralized with 20% sodium hydroxide aqueous solution until the aqueous phase reached about pH 11 whilst the temperature was controlled at 0°C to 15°C.

After liquid separation was performed, the aqueous phase was extracted, and the combined organic phase was washed with water, dried, and concentrated under reduced pressure at 30°C to 45°C. Finally, the residue was crystallized with methyl tert-butyl ether to obtain a white solid, which was dried under reduced pressure at 40°C to 50°C to obtain compound 1C, i.e., 1-(1-(azetidin-3-yl)piperidin-4-yl)-3-(4-phenoxyphenyl)-1H-pyrazolo[3,4-d]pyrimidin-4-amine (76.3 g, yield: 86.9%, purity: 98.4%).

(3) Synthesis of compound 1E (i.e., tert-butyl 3-(4-(4-amino-3-(4-phenoxyphenyl)-1H-pyrazolo[3,4-d]pyrimidin-1-yl)piperidin-1-yl)-[1,3']-biazetidine-1'-carboxylate)

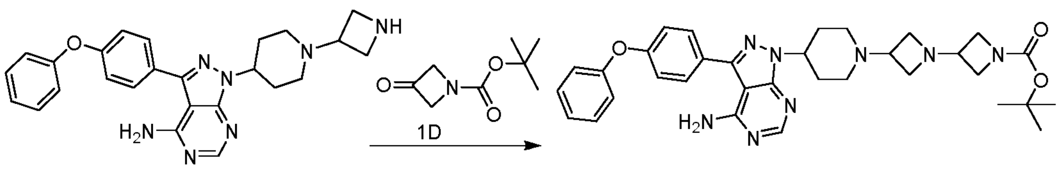


1C 1E

To a 2 L three-necked flask equipped with a thermometer and provided with mechanical stirring, dichloromethane (600 g) and compound 1C (99.8 g, 0.226 mol) were added, and then at around 25°C, tert-butyl 3-oxoazetidine-1-carboxylate (1D) (85.1 g, 0.497 mol), formic acid (28.5 g, 0.62 mol), and anhydrous sodium sulphate (90.0 g, 0.634 mol) were added. After the mixture was stirred for 1 h, sodium triacetoxyborohydride (100.0 g, 0.472 mol) was slowly added in portions. Upon completion of addition, the resulting mixture was reacted at 25°C to 35°C for about 3 to 5 hours.

After the reaction was completed, the reaction solution was filtered, and the resulting filtrate was neutralized with 15% sodium hydroxide aqueous solution until the aqueous phase reached about pH 11. After liquid separation was performed, the aqueous phase was extracted, and the combined organic phase was washed with water, dried, and concentrated under reduced pressure at around 40°C to obtain a yellow viscous substance, which was crystallized with methyl tert-butyl ether to obtain a white solid. The white solid was dried under reduced pressure at 40°C to 50°C to obtain compound 1E, i.e., tert-butyl-1-carboxylate-3-(4-(4-amino-3-(4-phenoxyphenyl)-1H-pyrazolo[3,4-d]pyrimidin-1-yl)piperidin-1-yl)-[1,3'-biazetidine (112.0 g, yield: 83.0%, purity: 89.2%).

(4) Synthesis of compound 1F-1 (i.e., 1-(1-([1,3'-diazepane]-3-yl)piperidin-4-yl)-3-(4-phenoxyphenyl)-1H-pyrazoline[3,4-d]pyrimidin-4-amine)

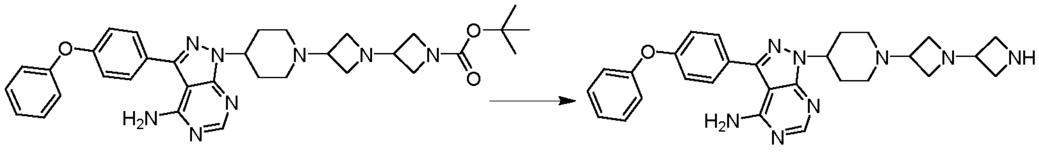

1E 1F-1

To a 2 L three-necked flask equipped with a thermometer and provided with mechanical stirring, dichloromethane (320 g) and compound 1E (69.8 g, 0.117 mol) were successively added with stirring. After the mixture was dissolved to yield a clear solution, trifluoroacetic acid (199.5 g, 1.75 mol) was added whilst the temperature was controlled at 15°C to 35°C. The resulting mixture was reacted at 25°C to 35°C for about 3 hours.

After the reaction was completed, the reaction solution was concentrated under reduced pressure at 30°C to 45°C to obtain a light yellow viscous substance. Then the light yellow viscous substance was dissolved with dichloromethane (300 g) and the resulting solution was neutralized with 20% sodium hydroxide aqueous solution until the aqueous phase reached about pH 11 whilst the temperature was controlled at 0°C to 15°C.

After liquid separation was performed, the aqueous phase was extracted, and the combined organic phase was washed with water, dried, and concentrated under reduced pressure at 30°C to 45°C. Finally, the residue was crystallized with methyl tert-butyl ether to obtain a white solid, which was dried under reduced pressure at 40°C to 50°C to obtain compound 1F-1, i.e., 1-(1-([1,3'-diazepane]-3-yl)piperidin-4-yl)-3-(4-phenoxyphenyl)-1H-pyrazoline[3,4-d]pyrimidin-4-amine (48.0 g, yield: 82.6%, purity: 90.1%).

(5) Synthesis of compound 1 (i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2, 6-dioxo-3-piperidyl)isoindoline-1,3 -dione)

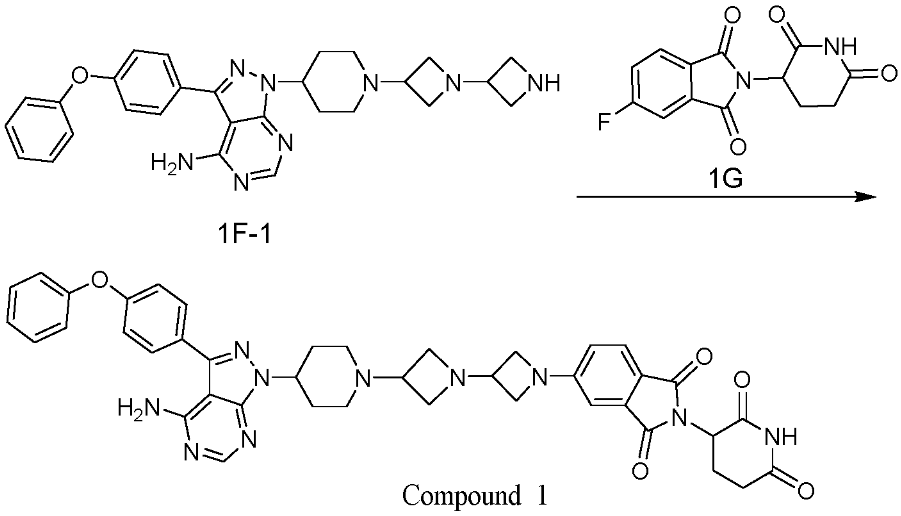

1F-1

1G

Compound 1

At around 20°C, to a 2 L three-necked flask equipped with a thermometer and provided with mechanical stirring, dimethylsulphoxide (150 g), compound 1F-1 (35.5 g, 0.0715 mol), and N,N-diisopropylethylamine (42.6 g, 0.33 mol) were successively added with stirring to form a solution. The solution was then added to a 60°C to 70°C solution containing dimethylsulphoxide (300 g) and compound 1G (24.0 g, 0.087 mol), and the resulting mixture was reacted at 85°C to 90°C for 6 to 8 hours.

After the reaction was completed, the reaction solution was cooled to 15°C to 25°C. Liquid separation was performed to remove the upper layer, which contains the vast majority of N,N-diisopropylethylamine. The reaction solution in the lower layer was poured into water (3 L) for crystallization. After filtering, the resulting mixture was slurried with

anhydrous ethanol (0.5 L) for 1 hour and then filtered to obtain compound 1 as a yellow solid, i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione (38.2 g, yield: 71.0%, purity: 84.5%).

(6) Synthesis of compound 1-1 (i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione dimaleate)

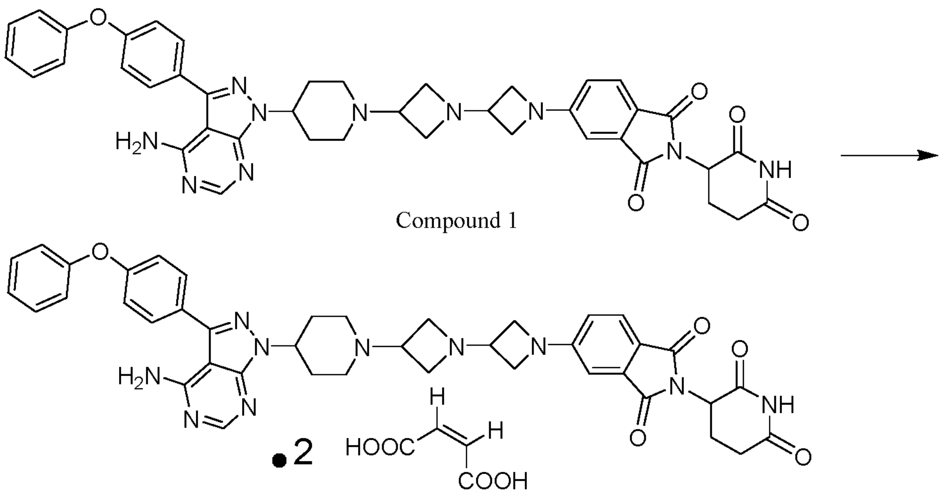

Compound 1

Compound 1-1

At around 20°C, to a 2 L three-necked flask equipped with a thermometer and provided with mechanical stirring, dichloromethane (1200 g), methanol (30 g), and compound 1 (70.1 g, 0.09 mol) were successively added with stirring. Then a solution containing methanol (45 g) and maleic acid (35.2 g, 0.3 mol) was added at 10°C to 20°C. Subsequently, the reaction solution was cooled to around 0°C for crystallization for 4 hours.

After filtering, the resulting mixture was dried under reduced pressure at 40°C to 50°C to obtain a crude, which was further refined and purified to obtain compound 1-1 as a yellow solid, i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione dimaleate (53.2 g, yield: 60.0%, purity: 99.2%).

Example 3

(1) Synthesis of compound 1B (i.e., tert-butyl 3-(4-(4-amino-3-(4-phenoxyphenyl)-1H-pyrazolo[3,4-d]pyrimidin-1-yl)piperidin-1-yl)azetidine-1-carboxylate)

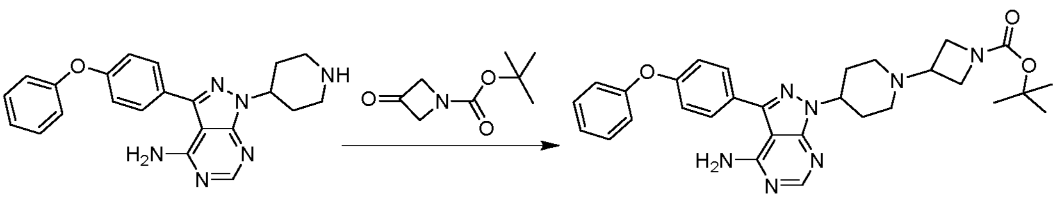

**1A** 1B

To a 2 L three-necked flask equipped with a thermometer and provided with mechanical stirring, dichloromethane (400 g) was added, and then at around 25°C, compound 1A (69.9 g, 0.181 mol), tert-butyl 3-oxoazetidine-1-carboxylate (62.3 g, 0.364 mol), formic acid (18.3 g, 0.398 mol), and anhydrous magnesium sulphate (61.0 g, 0.507 mol) were successively added with stirring. After the mixture was stirred for 1 h, sodium cyanoborohydride (27.3 g, 0.435 mol) was slowly added in portions. Upon completion of addition, the mixture was reacted at 25°C to 35°C for about 3 to 5 hours.

After the reaction was completed, the reaction solution was filtered, and the resulting filtrate was neutralized with 15% sodium hydroxide aqueous solution until the aqueous phase reached about pH 10. After liquid separation was performed, the aqueous phase was extracted, and the combined organic phase was washed with water, dried, and concentrated under reduced pressure at around 40°C to obtain a yellow viscous substance, which was crystallized with methyl tert-butyl ether to obtain a white solid. The white solid was dried under reduced pressure at 40°C to 50°C to obtain compound 1B, i.e., tert-butyl 3-(4-(4-amino-3-(4-phenoxyphenyl)-1H-pyrazolo[3,4-d]pyrimidin-1-yl)piperidin-1-yl)azetidine-1-carboxylate (94.4 g, yield: 96.2%, purity: 98.9%).

(2) Synthesis of compound 1C (i.e., 1-(1-(azetidin-3-yl)piperidin-4-yl)-3-(4-phenoxyphenyl)-1H-pyrazolo[3,4-d]pyrimidin-4-amine)

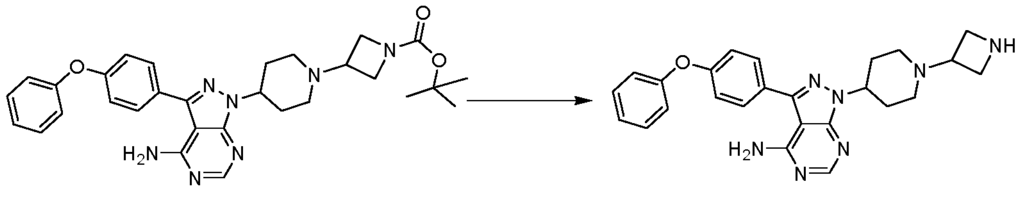

1B

1C

To a 2 L three-necked flask equipped with a thermometer and provided with mechanical stirring, methanol (539 g) and compound 1B (80.2 g, 0.148 mol) were successively added with stirring. After the mixture was dissolved to yield a clear solution, concentrated hydrochloric acid (30% mass concentration, 270.5 g, 2.223 mol) was added whilst the temperature was controlled at 10°C to 15°C. The resulting mixture was reacted at 25°C to 35°C for 2 to 3 hours.

After the reaction was completed, the reaction solution was concentrated under reduced pressure at 30°C to 45°C to obtain a light yellow viscous substance. Then the light yellow viscous substance was dissolved with dichloromethane (500 g) and the resulting solution was neutralized with 20% sodium hydroxide aqueous solution until the aqueous phase reached about pH 9 whilst the temperature was controlled at 0°C to 15°C.

After liquid separation was performed, the aqueous phase was extracted, and the combined organic phase was washed with water, dried, and concentrated under reduced pressure at 30°C to 45°C. Finally, the residue was crystallized with methyl tert-butyl ether to obtain a white solid, which was dried under reduced pressure at 40°C to 50°C to obtain compound 1C, i.e., 1-(1-(azetidin-3-yl)piperidin-4-yl)-3-(4-phenoxyphenyl)-1H-pyrazolo[3,4-d]pyrimidin-4-amine (56.7 g, yield: 86.8%, purity: 98.1%).

(3) Synthesis of compound 1E (i.e., tert-butyl 3-(4-(4-amino-3-(4-phenoxyphenyl)-1H-pyrazolo[3,4-d]pyrimidin-1-yl)piperidin-1-yl)-[1,3']-biazetidine-1'-carboxylate)

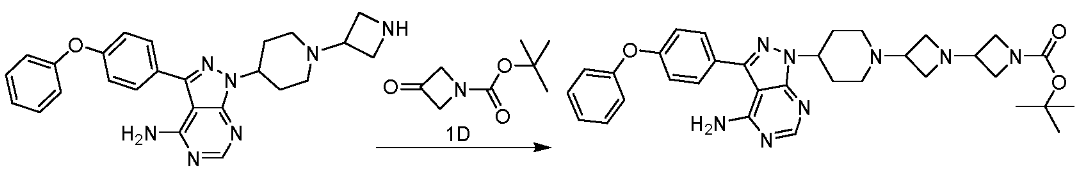


1C

1E

To a 2 L three-necked flask equipped with a thermometer and provided with mechanical stirring, dichloromethane (600 g) was added, and then at around 25°C, compound 1C (99.8 g, 0.226 mol), tert-butyl 3-oxoazetidine-1-carboxylate (1D) (85.6 g, 0.500 mol), acetic acid (36.0 g, 0.600 mol), and anhydrous sodium sulphate (100.0 g, 0.704 mol) were successively added with stirring. After the mixture was stirred for 1 h 1 h, sodium triacetoxyborohydride (106.0 g, 0.500 mol) was slowly added in portions. Upon completion of addition, the resulting mixture was reacted at 25°C to 30°C for 3 to 5 hours.

After the reaction was completed, the reaction solution was filtered, and the resulting filtrate was neutralized to pH 9-11with 15% sodium hydroxide aqueous solution. After liquid separation was performed, the aqueous phase was extracted, and the combined organic phase was washed with water, dried, and concentrated under reduced pressure

at around 40°C to obtain a yellow viscous substance, which was crystallized with methyl tert-butyl ether to obtain a white solid. The white solid was dried under reduced pressure at 40°C to 50°C to obtain compound 1E, i.e., tert-butyl-1-carboxylate-3-(4-(4-amino-3-(4-phenoxyphenyl)-1H-pyrazolo[3,4-d]pyrimidin-1-yl)piperidin-1-yl)-[1,3'-biazetidine (112.0 g, yield: 83.0%, purity: 89.3%).

(4) Synthesis of compound 1F-1 (i.e., 1-(1-([1,3'-diazepane]-3-yl)piperidin-4-yl)-3-(4-phenoxyphenyl)-1H-pyrazoline[3,4-d]pyrimidin-4-amine)

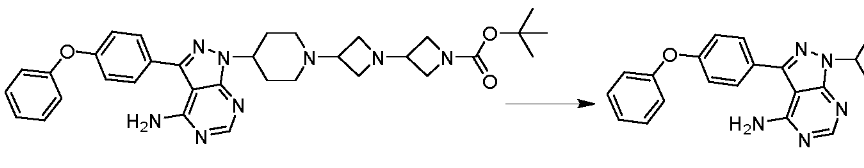

1E 1F-1

To a 2 L three-necked flask equipped with a thermometer and provided with mechanical stirring, methanol (300 g) and compound 1E (101.4 g, 0.17 mol) were successively added with stirring. After the mixture was dissolved to yield a clear solution, hydrochloric acid (30% mass concentration, 165.5 g, 1.36 mol) was added whilst the temperature was controlled at 15°C to 35°C. The resulting mixture was reacted at 25°C to 35°C for about 3 hours.

After the reaction was completed, the reaction solution was concentrated under reduced pressure at 30°C to 45°C to obtain a light yellow viscous substance. Then the light yellow viscous substance was dissolved with dichloromethane (300 g) and the resulting solution was neutralized with 20% sodium hydroxide aqueous solution until the aqueous phase reached about pH 11 whilst the temperature was controlled at 0°C to 15°C.

After liquid separation was performed, the aqueous phase was extracted, and the combined organic phase was washed with water, dried, and concentrated under reduced pressure at 30°C to 45°C. Finally, the residue was crystallized with methyl tert-butyl ether to obtain a white solid, which was dried under reduced pressure at 40°C to 50°C to obtain compound 1F-1, i.e., 1-(1-([1,3'-diazepane]-3-yl)piperidin-4-yl)-3-(4-phenoxyphenyl)-1H-pyrazoline[3,4-d]pyrimidin-4-amine (67.5 g, yield: 80.0%, purity: 90.3%).

(5) Synthesis of compound 1 (i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2, 6-dioxo-3-piperidyl)isoindoline-1,3 -dione)

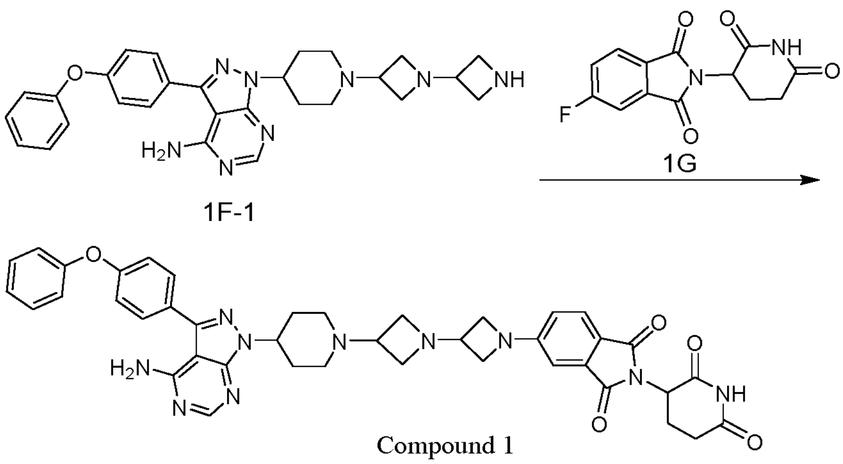

1G

1F-1

Compound 1

At around 20°C, to a 2 L three-necked flask equipped with a thermometer and provided with mechanical stirring, dimethylsulphoxide (150 g), compound 1F-1 (64.5 g, 0.13 mol), and triethylamine (33.4 g, 0.33 mol) were successively added with stirring to form a solution. The solution was then added to a 60°C to 70°C solution containing dimethylsulphoxide (300 g) and compound 1G (i.e., 2-(2,6-dioxopiperidin-3-yl)-5-fluoroisoindole-1,3-dione, CAS NO.: 835616-61-0) (55.2 g, 0.2 mol), and the resulting mixture was reacted at 85°C to 90°C for 6 to 8 hours.

After the reaction was completed, the reaction solution was cooled to 15°C to 25°C. Liquid separation was performed to remove the upper layer, which contains the vast majority of N,N-diisopropylethylamine. The reaction solution in the lower layer was poured into water (3 L) for crystallization. After filtering, the resulting mixture was slurried with anhydrous ethanol (1.0 L) for 1 hour and then filtered to obtain compound 1 as a yellow solid, i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione (68.5 g, yield: 70.0%, purity: 87.1%).

(6) Synthesis of compound 1-1 (i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione fumarate)

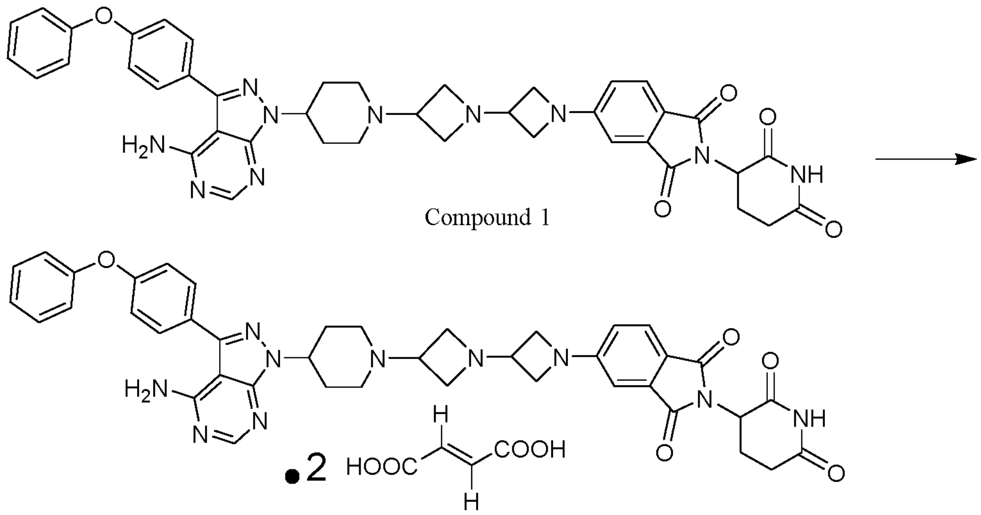

Compound 1

Compound 1-1

At around 20°C, to a 2 L three-necked flask equipped with a thermometer and provided with mechanical stirring, dichloromethane (1200 g), methanol (30 g), and compound 1 (70.1 g, 0.09 mol) were successively added with stirring. Then a solution containing methanol (45 g) and fumaric acid (34.8 g, 0.3 mol) was added. Subsequently, the reaction solution was cooled to around 0°C for crystallization for 3 hours.

After filtering, the resulting mixture was dried under reduced pressure at 40°C to 50°C to obtain a crude, which was further refined and purified to obtain compound 1-1 as a yellow solid, i.e., 5-[3-[3-[4-[4-amino-3-(4-phenoxyphenyl)]pyrazolo[3,4-d]pyrimidin-1-yl]-1-piperidyl]azetidin-1-yl]azetidin-1-yl]-2-(2,6-dioxo-3-piperidyl)isoindoline-1,3-dione difumarate (50.3 g, yield: 56.7%, purity: 99.2%).

$^1$HNMR (DMSO-d6): δ 11.09 (s, 1H), 8.27 (s, 1H), 7.70 ~ 7.66 (m, 3H), 7.47 ~ 7.43 (m, 2H), 7.22-7.13 (m, 5H), 6.87 (d, 1H), 6.74 ~ 6.71 (m, 1H), 6.16 (s, 4H), 5.10 ~ 5.05 (m, 1H), 4.97 ~ 4.92 (m, 1H), 4.20 ~ 4.17 (m, 2H), 4.05 (br, 1H), 3.99 ~ 3.97 (m, 2H), 3.87 (br, 2H), 3.70 (br, 3H), 3.32 ~ 3.30 (m, 2H), 2.94 ~ 2.84 (m, 1H), 2.87 (br, 2H), 2.62 ~ 2.54 (m, 2H), 2.44 ~ 2.35 (m, 2H), 2.14-2.11 (m, 2H), 2.04-2.01 (m, 1H).

(+)ESI-MS:753.3[m+1].

**Detection of BTK degradation in Mino cells**

Mino human mantle cell lymphoma cell line was purchased from ATCC and cultured under the following conditions: RPMI-1640 + 15% FBS + 1% double antibody in a 37°C, 5% $CO_2$ incubator. Cells were plated in a 6-well plate, with $5 \times 10^5$ cells/well. After plating, the compounds at different concentrations were added and cultured in an incubator at 37°C under 5% $CO_2$ for 48 hours. After culturing, the cells were collected, and RIPA lysis buffer (Beyotime, Cat. P0013B) was added. The cells were lysed on ice for 15 minutes and centrifuged at 12000 rpm at 4°C for 10 minutes. The protein sample of the supernatant was collected, and the protein was quantified by using the BCA kit (Beyotime, Cat. P0009), and then the protein was diluted to 0.25 mg/mL. The expressions of BTK (CST, Cat. 8547S) and the internal reference β-actin (CST, Cat. 3700S) were detected using a fully automated western blot quantitative analyzer (Proteinsimple) with a kit (Protein simple, Cat. SM-W004). The expression level of BTK relative to the internal reference was calculated using Compass software, and the $DC_{50}$ value was calculated using Origen9.2 software according to formula (1). Specifically, the BTK administration denoted the expression level of BTK in administration groups at different doses,

and the BTK vehicle denoted the expression level of BTK in the vehicle control group.

$$BTK\% = BTK\ administration/BTK\ vehicle \times 100 \qquad \text{formula (1)}$$

Table 1 $DC_{50}$ value of BTK degradation in Mino cells

| Serial No. | Compound No. | $DS_{50}$(nM) |
|---|---|---|
| 1 | Compound 1 | 10.9 |
| Conclusion: compound 1 and compound 2 had a significant degradation effect on BTK in Mino cells. | | |

**Detection of BTK protein degradation in spleen of mice**

Female ICR mice, 6-8 weeks old, were purchased from BEIJING VITAL RIVER LABORATORY ANIMAL TECHNOLOGY CO., LTD., and the experiment was started after 3 days of acclimatization. After intragastric administration of different doses of the compound for 3 consecutive days, the spleen of mice was taken, the spleen cells were collected, and RIPA lysis buffer (Beyotime, Cat. P0013B) was added. The cells were lysed on ice for 15 minutes and centrifuged at 12000 rpm at 4°C for 10 minutes. The protein sample of the supernatant was collected, the protein was quantified by using the BCA kit (Beyotime, Cat. P0009), and then the protein was diluted to 0.25 mg/mL. The expressions of BTK (CST, Cat. 8547S) and the internal reference β-actin (CST, Cat. 3700S) were detected using a fully automated western blot quantitative analyzer (Proteinsimple). The expression level of BTK relative to the internal reference was calculated using Compass software, and the $DD_{50}$ value was calculated using Origen9.2 software according to formula (2). Specifically, the $BTK_{administration}$ denoted the expression level of BTK in administration groups at different doses, and the $BTK_{vehicle}$ denoted the expression level of BTK in the vehicle control group.

$$BTK\% = BTK_{administration}/BTK_{vehicle} \times 100 \quad \text{formula (2)}$$

Table 2 $DD_{50}$ value of compound on BTK protein degradation in spleen of mice

| Serial No. | Compound No. | $DD_{50}$ (mg/kg) |
|---|---|---|
| 2 | Compound 1 | 3.8 |
| Conclusion: compound 1 and compound 2 had a significant degradation effect on BTK proteins in spleen of mice. | | |

***In vitro* kinase detection**

Kinases BTK wt (Carna, Cat. No. 08-180) and BTK C481S (Carna, Cat. No. 08-547) were prepared into a 2.5× kinase solution, and the substrates FAM-P2 (GL Biochem, Cat. No. 112394) and ATP (Sigma, Cat. No. A7699-1G) were prepared into a 2.5× substrate solution, respectively. 5 μL of compounds at different concentrations were added to a 384-well plate. 10 μL of 2.5× kinase solution was added, and the resulting mixture was incubated at room temperature for 10 min. 10 μL of 2.5× substrate solution was added, and the mixture was incubated at 28°C for an appropriate period of time. The reaction was stopped by adding 30 μL of stop buffer, and the detection was carried out by using Caliper EZ reader2. The $IC_{50}$ value was calculated by using XLFit excel add-in version 5.4.0.8 software. The calculation formula of the inhibition rate was shown in formula (3), wherein max denoted the readout of the DMSO control, min denoted the readout of the negative control, and conversion denoted the readout of the compound

$$\text{Inhibition rate\%} = (\text{max-conversion})/(\text{max-min})*100. \quad \text{formula (3)}$$

The results were as shown in Table 3:

Table 3 $IC_{50}$ value on BTK wt/C481S kinase inhibition

| Serial No. | Compound No. | BTK C481S $IC_{50}$ (nM) | BTK wt $IC_{50}$ (nM) |
|---|---|---|---|
| 1 | Compound 1 | 8 | 6.3 |
| Conclusion: compound 1 had a significant inhibitory effect on BTK wt/C481S kinase. | | | |

## Claims

1. A method for preparing compound (II) by the following reaction formula:

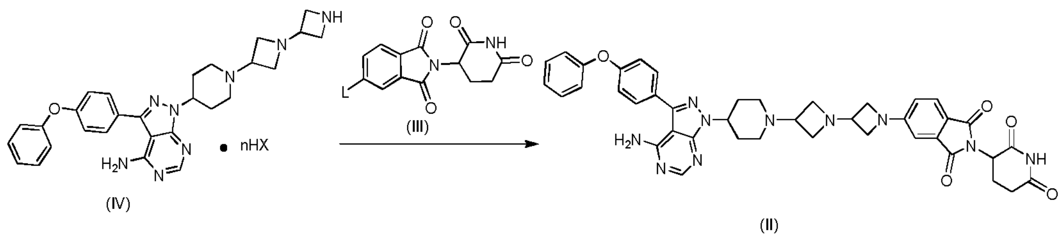


(IV) (III) (II)

wherein L is selected from a trifluoromethanesulphonate group, F, Cl, Br, I,

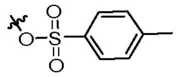, 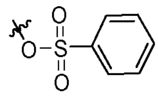, or 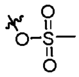;

HX is selected from acetic acid, hydrochloric acid, sulphuric acid, hydrobromic acid, hydroiodic acid or trifluoroacetic acid;
n is selected from 0, 1, 1.5, 2, 3 or 4;
compound (IV) is reacted with compound (III) in the presence of an alkaline reagent and a solvent to obtain the compound (II); and
when n = 0, i.e., the compound (IV) is in the form of a free base, the molar ratio of the alkaline reagent to the compound (IV) is ≤ 4.90 : 1.

2. The preparation method according to claim 1, wherein the alkaline reagent is selected from an organic amine reagent, preferably one or more of triethylamine, diethylamine or N,N-diisopropylethylamine.

3. The preparation method according to claim 1, wherein the solvent is selected from a polar aprotic solvent, preferably one or more of acetonitrile, N,N-dimethylformamide, N,N-diethylformamide, N,N-dimethylacetamide, dimethylsulphoxide or N-methyl-2-pyrrolidone.

4. The preparation method according to claim 1, wherein the reaction is performed at a temperature of 30°C-120°C, preferably 60°C-110°C, and more preferably 80°C-100°C.

5. The preparation method according to claim 1, comprising: reacting the compound (II) with HY to prepare compound (I),

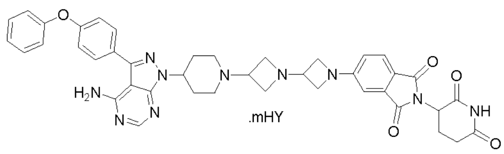


(I)

wherein HY is selected from a pharmaceutically acceptable salt, preferably fumaric acid, formic acid, acetic acid, butanedioic acid, hydrochloric acid, sulphuric acid, tartaric acid, p-methylbenzoic acid, methanesulphonic

acid, malic acid, maleic acid and succinic acid; and
m is selected from 0.5, 1, 1.5, 2 or 3.

**6.** The preparation method according to claim 5, wherein the solvent in the reaction of the compound (II) with HY is selected from one of or a mixed solvent of two or more of an alkane solvent, a halogenated alkane solvent, an alcohol solvent, a ketone solvent, an ester solvent, an ether solvent, a nitrile solvent and water.

**7.** The preparation method according to claim 5, wherein the solvent in the reaction of the compound (II) with HY is selected from one or more of dichloromethane, 1,2-dichloroethane, ethyl acetate, acetone, methanol, ethanol, ethylene glycol, polyethylene glycol, isopropanol, diethyl ether, tetrahydrofuran and water.

**8.** A method for preparing compound (IV) or compound (VI-1) by the following reaction formulas:

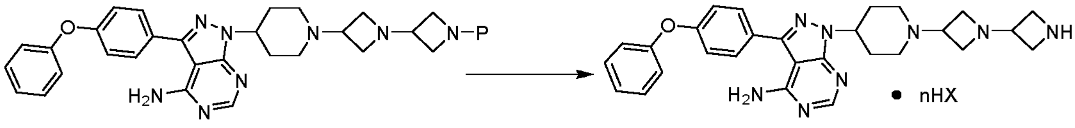

(V) (IV)

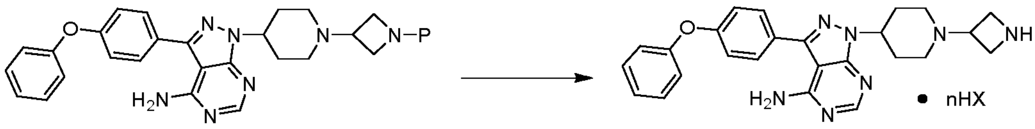

(VII) (VI-1)

wherein P is selected from an amino protecting group, preferably tertbutoxycarbonyl, benzyloxycarbonyl, methoxycarbonyl, ethoxycarbonyl, p-toluenesulphonyl, trifluoroacetyl, triphenylmethyl and p-methoxybenzyl;
HX is selected from acetic acid, sulphuric acid, hydrobromic acid, hydroiodic acid or trifluoroacetic acid;
n is selected from 0, 1, 1.5, 2, 3 or 4;
compound (V) is reacted in the presence of an acidic reagent HX to obtain the compound (IV);
and compound (VII) is reacted in the presence of an acidic reagent HX to obtain the compound (VI-1).

**9.** The preparation method according to claim according to claim 8, wherein the reaction involves a solvent selected from a polar protic solvent, a polar aprotic solvent or a mixture thereof, preferably one or more of methanol, ethanol, water, dichloromethane, 1,2-dichloroethane, chloroform, carbon tetrachloride or tetrahydrofuran.

**10.** The preparation method according to claim according to claim 8, wherein the reaction is performed at a temperature of optionally 0°C-60°C, preferably 10°C-40°C.

**11.** A method for preparing compound (IV) or compound (VI-1) by the following reaction formulas:

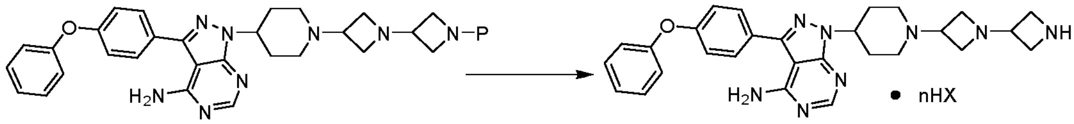

(V) (IV)

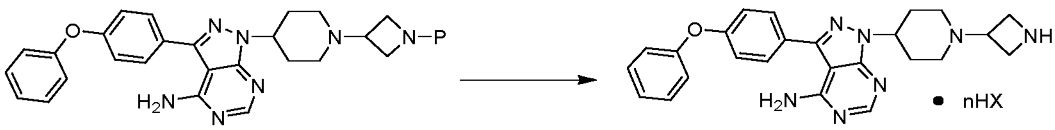

(VII) (VI-1)

wherein P is selected from an amino protecting group, preferably tertbutoxycarbonyl, benzyloxycarbonyl, methoxycarbonyl, ethoxycarbonyl, p-toluenesulphonyl, trifluoroacetyl, triphenylmethyl and p-methoxybenzyl;
HX is selected from hydrochloric acid;
n is selected from 0, 1, 1.5, 2, 3 or 4;
compound (V) is reacted in the presence of hydrochloric acid and a polar protic solvent to obtain the compound (IV); and
compound (VII) is reacted in the presence of hydrochloric acid and a polar protic solvent to obtain the compound (VI-1).

**12.** The preparation method according to claim 11, wherein the solvent is selected from a polar protic solvent, preferably one or more of methanol, ethanol and water, a polar aprotic solvent is optionally further added to the reaction, the polar aprotic solvent is preferably one or more of dichloromethane, 1,2-dichloroethane, chloroform, carbon tetrachloride or tetrahydrofuran, and the reaction is performed at a temperature of preferably 0°C-60°C, more preferably 10°C-40°C.

**13.** A method for preparing compound (V) or (VII) by the following reaction formulas:

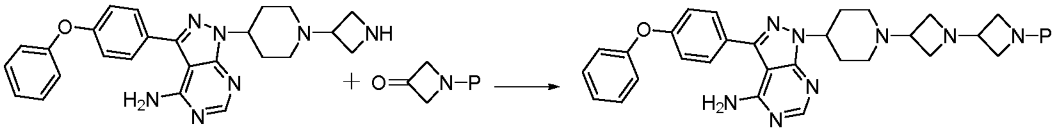

1a

(VI) (V)

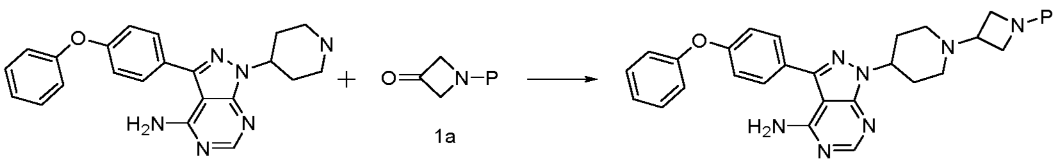

1a

(VIII) (VII)

wherein P is selected from an amino protecting group, preferably tertbutoxycarbonyl, benzyloxycarbonyl, methoxycarbonyl, ethoxycarbonyl, p-toluenesulphonyl, trifluoroacetyl, triphenylmethyl and p-methoxybenzyl;
compound (VI) is reacted with 1a in the presence of an acidic reagent and a reducing agent, optionally with the addition of a desiccant, and then subjected to a work-up to obtain the compound (V), and the reaction is performed at a temperature of preferably 0°C-40°C, more preferably 20°C-40°C;
and compound (VIII) is reacted with 1a in the presence of an acidic reagent and a reducing agent, optionally with the addition of a desiccant, and then subjected to a work-up to obtain the compound (VII), and the reaction is performed at a temperature of preferably 0°C-40°C, more preferably 20°C-40°C.

**14.** The preparation method according to claim 13, wherein the desiccant is selected from one or more of anhydrous sodium sulphate, anhydrous magnesium sulphate, anhydrous calcium sulphate or a molecular sieve.

**15.** The preparation method according to claim 13, wherein the reaction involves a solvent selected from a polar aprotic solvent, preferably one or more of 1,2-dichloroethane, chloroform or dichloromethane;

the acidic reagent is optionally one or more of hydrochloric acid, acetic acid, formic acid, propionic acid, butyric acid, sulphuric acid, hydrobromic acid, hydroiodic acid or trifluoroacetic acid; and
the reducing agent is selected from a boron reducing agent, preferably one or more of sodium borohydride, sodium triacetoxyborohydride, sodium triethylborohydride, sodium cyanoborohydride, potassium borohydride or lithium borohydride.

**16.** The preparation method according to claim 13, wherein the work-up comprises: adjusting a reaction system to a neutral to weakly basic pH, extracting, and concentrating an organic phase to obtain the compound (V) or (VII).

**17.** The preparation method according to claim 16, wherein the work-up further comprises: crystallizing or/and slurrying

with a solvent, preferably methyl tert-butyl ether or diethyl ether, filtering, and drying a filter cake.

**18.** A method for preparing compound (I), the method comprising the following steps:

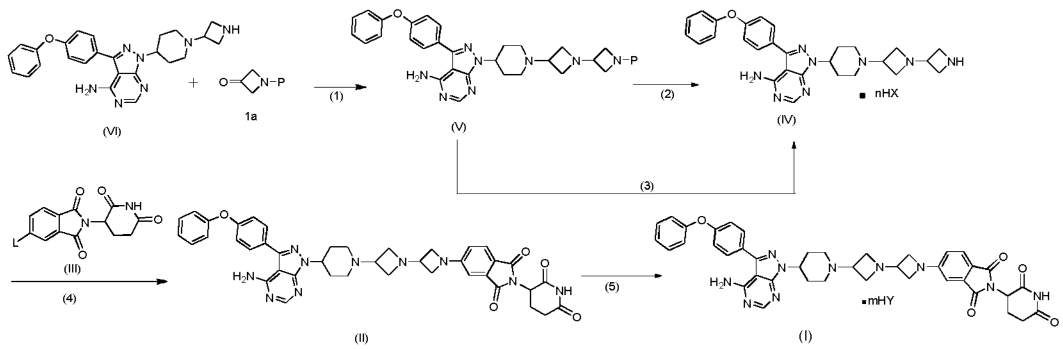


wherein L is selected from a trifluoromethanesulphonate group, F, Cl, Br, I,

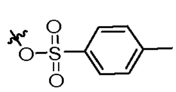, 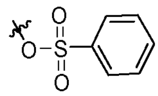, or 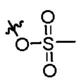;

HX is selected from acetic acid, hydrochloric acid, sulphuric acid, hydrobromic acid, hydroiodic acid or trifluoroacetic acid;
n is selected from 0, 1, 1.5, 2, 3 or 4;
HY is selected from a pharmaceutically acceptable salt, preferably fumarate, formate, acetate, butanedioate, hydrochloride, sulphate, tartrate, p-methylbenzoate, methanesulphonate, malate, maleate and succinate;
m is selected from 0.5, 1, 1.5, 2 or 3;
in step (1), compound (VI) is reacted with 1a in the presence of an acidic reagent and a reducing agent, optionally with the addition of a desiccant, and then subjected to a work-up to obtain compound (V);
in step (2), the compound (V) is reacted in the presence of an acidic reagent HX to obtain compound (IV), wherein the HX is selected from acetic acid, sulphuric acid, hydrobromic acid, hydroiodic acid or trifluoroacetic acid;
in step (3), the compound (V) is reacted in the presence of hydrochloric acid and a polar protic solvent to obtain the compound (IV), wherein the HX is HCl;
in step (4), the compound (IV) is reacted with compound (III) in the presence of an alkaline reagent to obtain compound (II); and
in step (5), the compound (II) is reacted with HY to prepare the compound (I).

**19.** The preparation method according to claim 18, wherein

in step (1), the acidic reagent is selected from one or more of hydrochloric acid, acetic acid, formic acid, propionic acid, butyric acid, sulphuric acid, hydrobromic acid, hydroiodic acid or trifluoroacetic acid, the reducing agent is selected from a boron reducing agent, preferably one or more of sodium borohydride, sodium triacetoxyborohydride, sodium triethylborohydride, sodium cyanoborohydride, potassium borohydride or lithium borohydride, and the desiccant is selected from one or more of anhydrous sodium sulphate, anhydrous magnesium sulphate, anhydrous calcium sulphate or a molecular sieve;
and the alkaline reagent in step (4) is selected from an organic amine reagent, preferably one or more of triethylamine, diethylamine or N,N-diisopropylethylamine.

**20.** The preparation method according to claim 18, wherein

step (1) involves a solvent selected from one or more of 1,2-dichloroethane, chloroform or dichloromethane;
step (2) involves a solvent selected from a polar aprotic solvent or a polar protic solvent, preferably one or more of dichloromethane, 1,2-dichloroethane, chloroform, carbon tetrachloride, tetrahydrofuran, methanol, ethanol and water;

in step (3), the solvent is selected from one or more of methanol, ethanol and water, a polar aprotic solvent is optionally further added to the reaction, and the polar aprotic solvent is preferably one or more of dichloromethane, 1,2-dichloroethane, chloroform, carbon tetrachloride or tetrahydrofuran;
step (4) involves a solvent selected from a polar aprotic solvent, preferably one or more of N,N-dimethylformamide, N,N-diethylformamide, N,N-dimethylacetamide, dimethylsulphoxide or N-methyl-2-pyrrolidone;
and step (5) involves a solvent selected from one or more of dichloromethane, 1,2-dichloroethane, ethyl acetate, acetone, methanol, ethanol, ethylene glycol, polyethylene glycol, isopropanol, diethyl ether, tetrahydrofuran and water.

**21.** The preparation method according to claim 18, wherein

the reaction in step (1) is performed at a temperature of 0°C-40°C, preferably 20°C-40°C;
the reaction in step (2) is performed at a temperature of 0°C-40°C, preferably 20°C-40°C;
the reaction in step (3) is performed at a temperature of 0°C-40°C, preferably 20°C-40°C;
the reaction in step (4) is performed at a temperature of 30°C-120°C, preferably 60°C-110°C, more preferably 80°C-100°C;
and the reaction in step (5) is performed at a temperature of 0°C-40°C, preferably 10°C-30°C.

**22.** A compound as represented below:

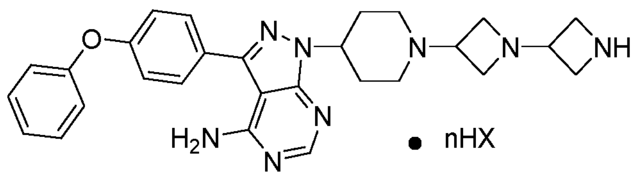

(IV)

wherein HX is selected from acetic acid, sulphuric acid, hydrobromic acid, hydroiodic acid or trifluoroacetic acid; and
n is selected from 1, 1.5, 2, 3 or 4.

**23.** A method for refining a compound as represented by formula (II), the method comprising mixing the compound as represented by formula (II) with one or more solvents selected from methanol, ethanol, isopropanol, ethyl acetate, acetone, methyl tert-butyl ether, diethyl ether or water, and crystallizing or/and slurrying the resulting mixture,

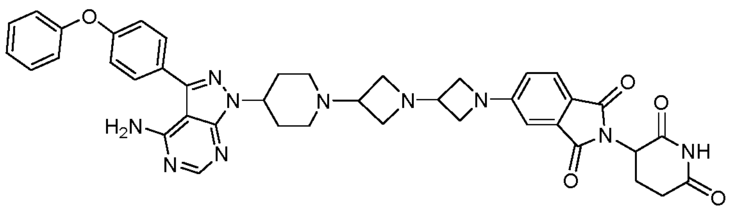

(II)

.

**24.** A method for refining a compound as represented by formula (IV), the method comprising mixing the compound as represented by formula (IV) with one or more solvents selected from methanol, ethanol, isopropanol, ethyl acetate, acetone, methyl tert-butyl ether, diethyl ether or water, and crystallizing or/and slurrying the resulting mixture,

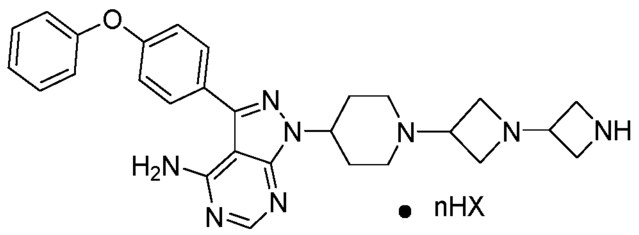

(IV)

wherein HX is selected from hydrochloric acid, acetic acid, sulphuric acid, hydrobromic acid, hydroiodic acid or trifluoroacetic acid; and
n is selected from 0, 1, 1.5, 2, 3 or 4.

**25.** A method for refining a compound as represented by formula (V) or (VII), the method comprising mixing the compound as represented by formula (V) or (VII) with one or more solvents selected from methanol, ethanol, isopropanol, ethyl acetate, acetone, methyl tert-butyl ether, diethyl ether or water, and crystallizing or/and slurrying the resulting mixture,

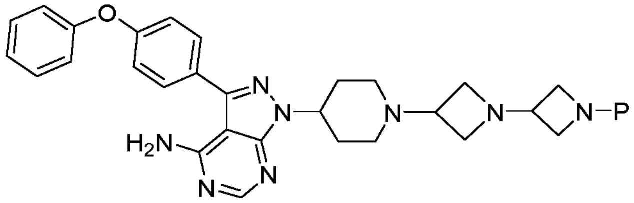

(V)

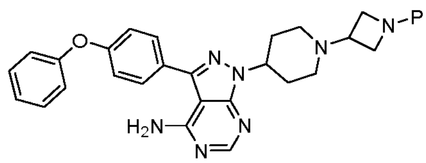

(VII)

wherein P is selected from an amino protecting group, preferably tertbutoxycarbonyl, benzyloxycarbonyl, methoxycarbonyl, ethoxycarbonyl, p-toluenesulphonyl, trifluoroacetyl, triphenylmethyl and p-methoxybenzyl.

**INTERNATIONAL SEARCH REPORT**

International application No.
**PCT/CN2021/132330**

**A. CLASSIFICATION OF SUBJECT MATTER**

C07D 487/04(2006.01)i; C07D 403/00(2006.01)i

According to International Patent Classification (IPC) or to both national classification and IPC

**B. FIELDS SEARCHED**

Minimum documentation searched (classification system followed by classification symbols)

C07D487/-; C07D403/-

Documentation searched other than minimum documentation to the extent that such documents are included in the fields searched

Electronic data base consulted during the international search (name of data base and, where practicable, search terms used)

WPI, EPODOC, CNPAT, REGISTRY(STN), CAPLUS(STN), CNKI: 四川海思科制药有限公司, 吡唑并嘧啶, 吡唑, 嘧啶, 哌啶, 氮杂环丁烷, 邻苯二甲酰亚胺, 布鲁顿酪氨酸蛋白激酶, pyrazolopyrimidinyl, pyrazol, pyrimidin+, piperidin+, azetidin+, phthalimid+, BTK, STN structure search

**C. DOCUMENTS CONSIDERED TO BE RELEVANT**

| Category* | Citation of document, with indication, where appropriate, of the relevant passages | Relevant to claim No. |
|---|---|---|
| A | CN 102656173 A (PHARMACYCLICS, INC.) 05 September 2012 (2012-09-05) embodiments 1-5 | 1-25 |
| A | WO 2017134685 A2 (SUN PHARMA ADVANCED RES. CO. LTD.) 10 August 2017 (2017-08-10) description SCHEME 9-10 | 1-25 |
| PX | WO 2020239103 A1 (SICHUAN HAISCO PHARMACEUTICAL CO., LTD.) 03 December 2020 (2020-12-03) embodiment 17 | 1-25 |
| PX | CN 112812100 A (SICHUAN HAISCO PHARMACEUTICAL CO., LTD.) 18 May 2021 (2021-05-18) Embodiment 3 | 1-25 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 January 2022** | **23 February 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2021/132330**

| Patent document cited in search report | Publication date (day/month/year) | Patent family member(s) | Publication date (day/month/year) |
|---|---|---|---|
| CN 102656173 A | 05 September 2012 | JP 2013507448 A | 04 March 2013 |
| | | JP 5717109 B2 | 13 May 2015 |
| | | EP 2650294 A1 | 16 October 2013 |
| | | EP 2650294 B1 | 20 July 2016 |
| | | US 7718662 B1 | 18 May 2010 |
| | | CL 2012000917 A1 | 13 July 2012 |
| | | CA 2776543 A1 | 21 April 2011 |
| | | KR 20120097376 A | 03 September 2012 |
| | | WO 2011046964 A2 | 21 April 2011 |
| | | WO 2011046964 A3 | 25 August 2011 |
| | | JP 2015134802 A | 27 July 2015 |
| | | ZA 201202552 B | 27 November 2013 |
| | | CO 6531429 A2 | 28 September 2012 |
| | | CN 102656173 B | 16 March 2016 |
| | | NZ 599396 A | 28 March 2014 |
| | | BR 112012008624 A2 | 13 June 2017 |
| | | ES 2590905 T3 | 24 November 2016 |
| | | ES 2590905 T8 | 09 December 2020 |
| | | US 2014080844 A1 | 20 March 2014 |
| | | US 7741330 B1 | 22 June 2010 |
| | | EP 2393816 A2 | 14 December 2011 |
| | | EP 2393816 A4 | 14 December 2011 |
| | | EA 201270553 A1 | 28 September 2012 |
| | | EA 021715 B1 | 31 August 2015 |
| | | IN 3012DEN2012 A | 31 July 2015 |
| | | IL 219076 D0 | 28 June 2012 |
| | | IL 219076 A | 30 June 2016 |
| | | SG 10201506776 R A | 29 October 2015 |
| | | US 2011086866 A1 | 14 April 2011 |
| | | US 9012463 B2 | 21 April 2015 |
| | | AU 2010306921 A1 | 10 May 2012 |
| | | AU 2010306921 B2 | 13 August 2015 |
| | | AU 2010306921 C1 | 17 March 2016 |
| | | MX 2012004258 A | 29 May 2012 |
| | | CR 20120244 A | 03 January 2013 |
| WO 2017134685 A2 | 10 August 2017 | WO 2017134685 A3 | 28 June 2018 |
| WO 2020239103 A1 | 03 December 2020 | AU 2020281410 A1 | 07 October 2021 |
| | | TW 202110825 A | 16 March 2021 |
| | | SG 11202110085 T A | 28 October 2021 |
| | | CN 113544130 A | 22 October 2021 |
| | | CA 3133004 A1 | 03 December 2020 |
| CN 112812100 A | 18 May 2021 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2020093455 W